# EUROPEAN PATENT SPECIFICATION

(11) **EP 1 968 632 B1**
(45) Date of publication and mention of the grant of the patent: **11.04.2012**
(21) Application number: 06821622.5
(22) Date of filing: 06.12.2006
(51) Int. Cl.: A61K 39/145

(54) **IMPROVED INFLUENZA VACCINE**
VERBESSERTER GRIPPEIMPFSTOFF
VACCIN AMELIORE CONTRE LA GRIPPE

(30) Priority: 06.12.2005 US 742574 P; 06.12.2005 US 742530 P
(43) Date of publication of application: 17.09.2008
(73) Proprietor: YEDA RESEARCH AND DEVELOPMENT CO., LTD., 76100 Rehovot (IL)
(72) Inventor: ARNON, Ruth, 76100 Rehovot (IL); BEN-YEDIDIA, Tamar, 76804 Markeret Batya (IL)
(74) Representative: Becker Kurig Straus
(86) International application number: PCT/IL2006/001403
(87) International publication number: WO 2007/066334

(56) References cited:
- WO-A-00/32228
- WO-A2-02/00885
- LEVI R ET AL: "Synthetic recombinant influenza vaccine induces efficient long-term immunity and cross-strain protection" VACCINE, BUTTERWORTH SCIENTIFIC. GUILDFORD, GB, vol. 14, no. 1, January 1996 (1996-01), pages 85-92, XP004057376 ISSN: 0264-410X
- CHEN Z ET AL: "Enhanced protection against a lethal influenza virus challenge by immunization with both hemagglutinin- and neuraminidase-expressing DNAs" VACCINE, BUTTERWORTH SCIENTIFIC. GUILDFORD, GB, vol. 17, no. 7-8, 26 February 1999 (1999-02-26), pages 653-659, XP004154803 ISSN: 0264-410X
- JEON S H ET AL: "Intranasal immunization with synthetic recombinant vaccine containing multiple epitopes of influenza virus" VACCINE, BUTTERWORTH SCIENTIFIC. GUILDFORD, GB, vol. 20, no. 21-22, 21 June 2002 (2002-06-21), pages 2772-2780, XP004357801 ISSN: 0264-410X
- BEN-YEDIDIA TAMAR ET AL: "Towards an epitope-based human vaccine for influenza." HUMAN VACCINES 2005 MAY-JUN, vol. 1, no. 3, May 2005 (2005-05), pages 95-101, XP001249210 ISSN: 1554-8600

## Description

### FIELD OF THE INVENTION

The present invention relates generally to influenza vaccines for human and veterinary use. In particular, the present invention provides a vaccine able to elicit long term and cross-strain protection comprising a plurality of chimeric proteins comprising the following influenza virus peptide epitopes: a B-cell and CTL epitope (M1 2-12; SEQ ID NO:25 or 26); a B-cell epitope (HA 91-108; SEQ ID NO:48); a Th epitope (HA 307-319; SEQ ID NO:57); two CTL epitopes (NP 335-350; SEQ ID NO:67, and NP 380-393; SEQ ID NO:68); and a further B-cell epitope (HA 354-372; SEQ ID NO:80).

### BACKGROUND OF THE INVENTION

### Influenza

Influenza is a disease caused by viruses of three main subtypes, Influenza A, B and C, which are classified according to their antigenic determinants. The influenza virion consists of a single stranded RNA genome closely associated with a nucleoprotein (NP) and enclosed by a lipoprotein envelope lined by matrix protein (M1) and carrying two major surface glycoprotein antigens, haemagglutinin (HA) and neuraminidase (NA). The HA and NA glycoproteins are most susceptible to change; for example, there are 16 immune classes of HA and 9 different NA classes that provide the basis for the different influenza virus subtypes like H1N1 or H3N2. Influenza A virus has an additional transmembrane glycoprotein, M2, which is highly conserved between the different HN subtypes. The M2 gene encodes a protein having 96-97-amino-acids that is expressed as a tetramer on the virion cell surface. It is composed of about 24 extracellular amino acids, about 19 transmembrane amino acids, and about 54 cytoplasmic residues (Lamb et al, 1985).

Influenza A and B viruses are the most common causes of influenza in man. Influenza has an enormous impact on public health with severe economic implications in addition to the devastating health problems, including morbidity and even mortality. Infection may be mild, moderate or severe, ranging from asymptomatic through mild upper respiratory infection and tracheobronchitis to a severe, occasionally lethal, viral pneumonia.

Influenza viruses have two important immunological characteristics that present a challenge to vaccine preparation. The first concerns genetic changes that occur in the surface glycoproteins every few years, referred to as "antigenic drift". This antigenic change produces viruses that elude resistance elicited by existing vaccines. The second characteristic of great public health concern is that influenza viruses, in particular influenza A virus can exchange genetic material and merge. This process, known as "antigenic shift", results in new strains different from both parent viruses, which can be lethal pandemic strains. Avian influenza is an influenza A virus that has crossed the species barrier from birds to mammals, including humans.

### Avian Influenza

Most avian influenza (AI) strains are classified as low pathogenic avian influenza (LPAI) and cause few clinical signs in infected birds. In contrast, high pathogenic avian influenza (HPAI) strains cause a severe and extremely contagious illness and death among infected birds.

Humans are not commonly affected by avian flu, however, the epidemics of highly pathogenic avian influenza (HPAI) recently seen in poultry in Asia increase opportunities for human exposure and infection. Severe cases have been reported in the past and during the current epidemics in Asia. Recent highly pathogenic outbreaks have been caused by influenza A viruses of subtypes H5 and H7. Of the 15 avian influenza virus subtypes, H5N1 is of particular concern since it mutates rapidly and has a documented propensity to acquire genes from viruses infecting other animal species. Its ability to cause disease in humans is now well documented. Birds that survive infection excrete virus for at least 10 days, orally and in feces, thus facilitating further spread at live poultry markets and by migratory birds.

The epidemic of HPAI caused by H5N1, which began in mid-December 2003 in certain Asian countries has spread and poses a public health concern. The spread of infection in birds increases the opportunities for direct infection of humans. If more humans become infected over time, the likelihood also increases that humans, if concurrently infected with human and avian influenza strains, could serve as the host for the emergence of a novel subtype with sufficient human influenza genes to be easily transmitted from person to person. Such an event would mark the start of an influenza pandemic.

AI spread between birds occurs primarily by direct contact between healthy birds and infected birds, and through indirect contact with contaminated equipment and materials. The virus is excreted through the feces of infected birds and through secretions from the nose, mouth and eyes.

Contact with infected fecal material is the most common mode of bird-to-bird transmission. Wild ducks often introduce low pathogenicity viruses into domestic flocks raised on range or in open flight pens through fecal contamination. Within a poultry house, transfer of the HPAI virus between birds can also occur via airborne secretions. The spread of avian influenza between poultry premises almost always follows the movement of contaminated people and equipment. Transfer of eggs is also a potential means of AI transmission.

### Influenza Virus Antigens and Vaccine Production

Immunization towards influenza virus is limited by the antigenic variation of the virus and by the restriction of the infection to the respiratory mucous membranes. The influenza vaccines currently available are based either on whole inactive virus, or on antigenic determinants, of the surface proteins. HA is a strong immunogen and is the most significant antigen in defining the serological specificity of the different virus strains.

The HA molecule (75-80 kD) comprises a plurality of antigenic determinants, several of which are in regions that undergo sequence changes in different strains (strain-specific determinants) and others in regions which are conserved in many HA molecules (common determinants). Due to these changes, flu vaccines need to be modified at least every few years.

Many influenza antigens, and vaccines prepared therefrom, are known in the art. US Patent 4,474,757 discloses a vaccine against influenza virus infections consisting of a synthetic peptide corresponding to an antigenic fragment of HA attached to a suitable macromolecular carrier, such as polymers of amino acids or tetanus toxoid.

PCT International Publication WO 93/20846 to some of the inventors of the present invention teaches a synthetic recombinant vaccine against a plurality of different influenza virus strains comprising at least one recombinant protein comprising the amino acid sequence of flagellin and at least one amino acid sequence of an epitope of influenza virus HA or NP, or an aggregate of said chimeric protein. Following this approach, a synthetic recombinant anti-influenza vaccine based on three epitopes was found to be highly efficient in mice. The exemplified vaccines included flagellin chimeras comprising the HA 91-108 epitope, a B-cell epitope from the HA which is conserved in all H3 strains and elicits anti-influenza neutralizing antibodies, together with one or both T-helper or CTL NP epitopes (NP 55-69 and NP 147-158, respectively), which induce MHC-restricted immune responses. A vaccine comprising a combination of the three above mentioned chimeras was considered to afford the best protection to viral infection.

PCT application publication WO 00/32228 to some of the inventors of the present invention teaches a human synthetic peptide-based influenza vaccine comprising at least four epitopes of influenza virus, said influenza virus epitopes being reactive with human cells, said epitopes comprising:
(i) one B-cell haemagglutinin (HA) epitope; (ii) one T-helper haemagglutinin (HA) or nucleoprotein (NP) epitope that can bind to many HLA molecules; and (iii) at least two cytotoxic lymphocyte (CTL) nucleoprotein (NP) or matrix protein (M) epitopes that are restricted to the most prevalent HLA molecules in different human populations, in particular specific ethnic or racial groups. The influenza peptide epitopes can be expressed as recombinant Salmonella flagellin. That vaccine requires the cumbersome preparation of at least four chimeric polypeptides.

PCT Application Publication WO 2004/080403 and US Patent Application Publication US2004/0223976 provide a vaccine against disease caused by infection with influenza virus, and methods of vaccination. Each vaccine comprises a plurality of peptides derived from the M2 and/or HA proteins of influenza virus chemically conjugated to a carrier protein. The conjugation is between one terminus of the peptide and a reactive site of the carrier protein where the carrier protein is selected from the outer membrane protein complex of Neisseria meningitidis, tetanus toxoid, hepatitis B surface antigen or core antigen, keyhole limpet hemocyanin, rotavirus capsid protein, and the L1 protein of bovine or human papillomavirus VLP. That disclosure requires a plurality of M2 or HA peptide epitopes covalently bound to the outer surface of a carrier protein and neither suggests nor teaches a vaccine comprising a chimeric polypeptide.

PCT Application Publication WO 99/07839 relates to influenza antigens for use in vaccines wherein the vaccines are comprised of a fusion product of at least the extracellular part of M2 and a presenting carrier. The M2 fragment was fused to the amino terminus of the carrier protein in order to retain a free N-terminus of the M2-domain and in this way mimic the wild type structure of the M2 protein. Furthermore that invention is exemplified by way of a M2 fusion protein wherein the intact extracellular portion of M2 fragment is fused to the N-terminus of the hepatitis B virus core protein in order to mimic the wild type structure of the M2 protein in viral particles and on infected cells, where the free N-terminus extends in the extracellular environment. That application neither teaches nor suggests an isolated M2 epitope that is conformationally constrained.

International Patent Application Publication No. WO 99/07839 teaches an immunogenic extracellular portion of a M2 membrane protein of an influenza A virus fused to a presenting carrier, which can be selected from the amino terminus of the human Hepatitis B virus core protein, third complement protein fragment d (C3d), tetanus toxin fragment C or yeast Ty particles. Other non-peptidic presenting carriers are mentioned, yet that invention is exemplified only by genetic fusion products.

Slepushkin et al. (1995) describes protection of mice to influenza A challenge by vaccination with a recombinant M2 protein expressed in baculovirus and administered with Freund's adjuvant.

PCT Application Publication No. WO 98/23735 discloses an influenza vaccine for inducing a cell-mediated cytolytic immune response against an antigen in a mammal comprising a fusion product of an influenza antigen and a stress protein or heat shock protein as carrier. The influenza antigen is selected from hemagglutinin, nucleoprotein, neuraminidase, M1, M2, PB1, PB2, PA and a combination thereof. There is neither teaching nor suggestion of a vaccine combining a M epitope with a HA epitope.

Zou, et al. (2005) teach the extracellular M2 6-13 peptide and suggest that that sequence may be useful in the preparation of an influenza vaccine. Liu, et al (2005) disclose host specific epitopes within the extracellular M2 sequences, which may be useful for the preparation of a bivalent influenza vaccine. The relevant epitopes include the M2 10-20 sequence common to human, avian and swine influenza.

PCT Application Publication No. WO 94/26903 relates to human influenza matrix protein peptides able to bind to human MHC Class I molecules. That invention provides candidate peptide epitopes able to bind to the groove of MHC class I molecules. identified using the antigen processing defective cell line174.CEM T2 (T2). There is neither teaching nor suggestion of a vaccine comprising a combination of M peptide epitope with a HA peptide epitope.

Levi R. et al. ("Synthetic recombinant influenza vaccine induces efficient long-term immunity and cross-strain protection", Vaccine, Butterworth Scientific. Guildford, GB, Vol. 14, No. 1, January 1996, p. 85-92) discloses a synthetic peptide-based influenza vaccine comprising a mixture of three epitopes selected from one B-cell HA epitope, one T helper NP epitope and one CTL epitope. Levi et al. do not mention matrix or influenza B epitopes.

Chen Z. et al. ("Enhanced protection against a lethal influenza virus challenge by immunization with both hemagglutinin- and neuraminidase-expressing DNAs", Vaccine Butterworth Scientific. Guildford, GB, Vol. 17, No. 7-8, February 1999, p. 653-659) describe DNA vaccines encoding HA, NA, and M1 full-length proteins. Shorter peptides are not specified.

WO 02/00885 A2 relates to influenza virus-like-particles comprising the proteins HA, NA, M1 and M2. Said application does not disclose any short peptides.

Jeon S.H. et al. (" Intranasal immunization with synthetic recombinant vaccine containing multip0le epitopes of influenza virus", Vaccine, Butterworth Scientific. Guildford, GB, Vol. 20, No. 21-22, June 2002, p. 2772-2780) relates to intranasal immunization with a synthetic recombinant influenza vaccine containing multiple epitopes of the influenza virus. Jeon et al. do not disclose M1 epitopes and influenza type B epitopes.

Ben-Yedidia et al. ("Towards an epitope-based human vaccine for influenza", Human Vaccines, Vol. 1, No. 3, May 2005, pages 95-101) is a review paper on peptide-based human vaccines for influenza. Matrix protein epitopes are not mentioned therein.

It would be highly advantageous to have an influenza vaccine that could be administered once and confer protection for several years or even a lifetime by providing cross-protection against new strains of viruses.

There remains an unmet need for a vaccine useful in eliciting an immune response to a broad range of influenza subtypes that affords long term and cross-species protection, is both cost effective and readily produced, can be administered in a variety of forms and is useful for animal and human immunization.

### SUMMARY OF THE INVENTION

The present invention provides an influenza vaccine eliciting long-term and cross-subtype protection against infection with influenza A and influenza B viruses, including the avian influenza serotypes. An unexpectedly robust immune response to influenza A or B virus is elicited by a vaccine comprising chimeric proteins which comprise at least one influenza A matrix protein (M) peptide epitope and at least one influenza A or B haemagglutinin (HA) peptide epitope. A vaccine comprising a combination of a M and a HA epitope overcomes drawbacks of the known vaccines including the need for including epitopes that are restricted to HLA molecules associated with different racial or ethnic populations. The vaccine of the present invention elicits cross-racial efficacy and Asian and African populations react as well as Caucasians.

Additionally, a surprisingly effective immune response to influenza A or B virus is elicited by a vaccine comprising a chimeric protein comprising a M2 peptide epitope and a flagellin amino acid sequence wherein the M2 peptide epitope is embedded within the flagellin polypeptide sequence. This finding is unexpected in view of the hitherto known M2 fusion proteins and conjugates that comprise at least the entire M2 extracellular region and mimic the conformation of the entire extracellular domain of the M2 protein.

In one aspect the present invention provides a vaccine for immunization of a subject comprising a plurality of chimeric proteins comprising the influenza virus peptide epitopes:
M1 2-12 (SEQ ID NO:25 or 26);
HA 91-108 (SEQ ID NO:48);
HA 307-319 (SEQ ID NO:57);
NP 335-350 (SEQ ID NO:67);
NP 380-393 (SEQ ID NO:68); and
HA 354-372 (SEQ ID NO:80).

According to one embodiment the vaccine further comprises an adjuvant or an excipient.

According to another embodiment the vaccine is formulated for administration by a modality selected from the group consisting of intraperitoneal, subcutaneous, intranasal, intramuscular, oral, topical and transdermal delivery.

According to another aspect, the vaccine is for use in a method for eliciting an immune response and conferring protection against influenza virus in a subject. Preferably the immune response is elicited against avian influenza, influenza type A, influenza type B or a combination thereof.

According to another aspect of the present invention the vaccine is used for the preparation of a pharmaceutical agent for eliciting an immune response and conferring protection against influenza virus in a subject.

In some embodiments the vaccine protects against influenza A, including avian influenza. In other embodiments the vaccine comprises an influenza B epitope and elicits protection against influenza B. In another embodiment the vaccine comprises peptide epitopes that elicit protection to both influenza A and influenza B virus.

Routes of administration of the vaccine include, but are not limited to intraperitoneal, subcutaneous, intranasal, intramuscular, oral, topical and transdermal delivery. Preferred routes of administration include oral, intranasal (IN) and intramuscular (IM) administration. In one embodiment the vaccine is formulated for intranasal administration. In another embodiment the vaccine is formulated for intramuscular administration

It is to be explicitly understood that known compositions are to be excluded from the present invention.

Further embodiments and the full scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### BRIEF DESCRIPTION OF THE FIGURES

Figure 1 presents a graph showing interferon gamma (IFNγ) secretion from lymphocytes incubated with the respective peptide after the second and third immunization of HHD/HLA A2 transgenic (TG) mice.
Figures 2A-2C show lysis of target cells by NK (Natural Killer cells) derived from mice immunized with individual or combination epitopes following the second (2A) and third immunization (2B). Even after the second immunization, NK cells from the mice immunized with the combination of 6 epitopes (hexa-vaccine1) were able to lyse the target cells more efficiently than the cells from mice vaccinated with the native flagella (2C). Specific lysis was determined at different E:T ratios.
Figures 3A and 3B show the results of immunizing C57B1/6 mice with Hexa-vaccine2, consisting of recombinant flagella comprising the HA 91-108, HA 354-372, HA 307-319, NP 335-350, NP 380-393 and M2 1-18 peptide epitopes. Serum was tested following 3 immunizations and the specificity of antibody (Ab or Ig) against the whole H3N2 influenza virus (3A) and against the specific M2 1-18 peptide (3B) was determined.
Figure 4 shows binding of peptide epitopes to HLA-A2 on T2 cells: High and dose dependent binding was shown by the M1 3-11 peptide. Other tested peptides, NP 336-344, NP 380-388 and HA 307-319, also showed binding capacity, which was not dose dependent.
Figures 5A-5C show antibody protection elicited by the recombinant peptide epitopes. A significant elevation in antibody (Ab) titer, specific to each epitope was obtained with epitopes M1 2-12, NP 335-350 and HA 91-108 in mice immunized with the single relevant epitope.
Figure 6 shows the humoral immune dose response obtained following vaccination of mice with Hexa-vaccine1.
Figure 7 shows the humoral immune response to flagella following intranasal and intramuscular administration of the Hexa-vaccine1.
Figure 8 shows the results of virus titration. After three vaccinations with Hexa-vaccine1, mice were infected with a sub-lethal dose of influenza virus H3N2 strain (A/Texas/1/77). The lungs of the mice were removed 5 days later for titration of viral load. The titration was performed in fertilized eggs.
Figure 9 presents the IgE titer in the dosing experiment and in the experiment for evaluating the cellular response.
Figure 10 shows IgE concentration (ng/ml) in sera of HHD transgenic mice immunized intranasally (IN) with recombinant flagella expressing influenza epitopes.
Figure 11 shows fold increase of IgG titer to 3 different influenza strains in sera of NZW rabbits immunized intranasally three times with recombinant flagella expressing six influenza epitopes (Hexa-vaccine2).
Figure 12 depicts pharmacokinetic data. Maximum serum concentration of Hexa-vaccine1 was observed after 15 minutes (Tₘₐₓ). Half (T_{½}) of the total exposure quantity was obtained within 30 minutes post dosing. Protein could not be detected after 12 hours.

### DETAILED DESCRIPTION OF THE INVENTION

The present invention is based on the unexpected discovery that a vaccine comprising at least one M peptide epitope and at least one HA peptide epitope is able to elicit long-term and cross strain protective immunity to influenza.

### Definitions

For convenience, certain terms employed in the specification, examples and claims are described herein.

The term "antigen presentation" means the expression of antigen on the surface of a cell in association with major histocompatability complex class I or class II molecules (MHC-I or MHC-II) of animals or with the HLA-I and HLA-II of humans.

The term "immunogenicity" or "immunogenic" relates to the ability of a substance to stimulate or elicit an immune response. Immunogenicity is measured, for example, by determining the presence of antibodies specific for the substance. The presence of antibodies is detected by methods known in the art, for example using an ELISA assay.

Influenza epitopes can be classified as B-cell type, T-cell type or both B cell and T cell type, depending on the type of immune response they elicit. The definition of B cell or T cell peptide epitope is not unequivocal; for example, a peptide epitope can induce antibody production but at the same time that epitope can possess a sequence that enables binding to the human HLA molecule, rendering it accessible to CTLs, hence a dual B cell and T cell classification for that particular epitope. "CTL", "killer T cells" or "cytotoxic T cells" is a group of differentiated T cells that recognize and lyse target cells bearing a specific foreign antigen that function in defense against viral infection and cancer cells. "T helper cell" or "Th" is any of the T cells that when stimulated by a specific antigen release cytokines that promote the activation and function of B cells and killer T cells.

The term "recombinant flagellin" refers to a flagellin polypeptide comprising a peptide epitope embedded within its sequence. A recombinant flagellin is distinct from a classical fusion protein in that the peptide or protein being expressed in a fusion protein is fused to a carrier protein at either its N- or C-terminus, leaving the other terminus free and conformationally unrestrained.

"Amino acid sequence", as used herein, refers to an oligopeptide, peptide, polypeptide, or protein sequence, and fragment thereof, and to naturally occurring or synthetic molecules.

"Avian influenza" or "AI" refers to avian influenza virus that infect birds, including domestic and wild birds. The known avian influenza viruses belong to the H5, H7 and H9 virus subtypes. The avian influenza virus may belong to the low pathogenic (LPAI) or high pathogenic type (HPAI) and may or may not have undergone antigenic shift. Certain strains of avian flu, including H5N1, H7N3, H7N7 and H9N2, have been shown to infect mammals, including humans.

### Peptide Epitopes Useful in Preparing a Vaccine

Peptide epitopes derived from influenza proteins are useful in preparing the composition of the present invention. One compositions disclosed includes at least one peptide epitope derived from influenza A M1 or M2 proteins in combination with an influenza A or influenza B HA peptide epitope. It is to be noted that peptide epitopes listed herein are provided as for exemplary purposes only. The influenza virus proteins vary between isolates, thereby providing multiple variant sequences for each influenza protein.

The matrix protein M1 is a major structural component of the influenza virus particles and forms an inner layer of the lipid cell-derived envelope. Within the virion and in infected cells at late stages of the virus replication, the M1 protein associates with the viral ribonucleoproteins (vRNPs), which are composed of viral RNA molecules, multiple copies of the NP, and the three subunits of the viral polymerase holding the ends of the viral RNAs. The N-terminal domain of M1 refers to amino acids 1 to about amino acid 20 of the M1 protein.

The matrix protein M2 is a hydrogen ion channel resulting in dissociation of the matrix and nucleoprotein complex within vacuoles. This ion channel releases the genome enabling viral RNA to enter the nucleus of the infected cell and initiate viral replication. Therapeutic substances against influenza, such as amantadine and rimantadine act by blocking the M2 activity. Influenza B has a counterpart protein known as NB; although there is no sequence similarity they are both transmembrane proteins and may share similar function. The extracellular domain of the M2 protein which is a transmembrane protein of influenza A virus, is nearly invariant in all influenza A strains. The N-terminal domain of M2 refers to the amino acid sequence N-terminal to the transmembrane domain.

Table 1 provides an exemplary list of M1 and M2 peptide epitopes that may be chosen for preparation of chimeric proteins.

**Table 1. M1 and M2 peptide epitopes**

| Epitope Type* | Epitope Position | Amino Acid Sequence | Nucleotide Sequence | NCBI # |
|---|---|---|---|---|
| | M2 6-9 | EVET (SEQ ID NO:1) | GAAGTGGAAACC (SEQ ID NO:81) | ABJ15715.1 |
| Th | M2 1-15 | | | ABJ15715.1 |
| | M2 10-18 | PIRNEWGCR (SEQ ID NO:3) | | ABD59884 |
| | M2 8-15 | ETPIRNEWGC (SEQ ID NO:4) | | ABD59884 |
| | M2 10-20 | PIRNEWGCRCN (SEQ ID NO:5) | | ABD59884 |
| CTL | M2 3-11 | LLTEVETPI (SEQ ID NO:6) | | ABD59884 |
| CTL | M2 2-10 | SLLTEVETP (SEQ ID NO:7) | | ABD59884 |
| CTL | M2 2-11 | SLLTEVETPI (SEQ ID NO:8) | | ABD59884 |
| CTL | M2 4-11 | LTEVETPLT (SEQ ID NO:9) | | ABD59884 |
| Th | M2 1-15 | | | ABD59884 |
| Th | M2 1-18 | | | ABD59884 |
| Th | M2 1-15 | | | AAK49250 |
| Th | M2 1-15 | | | ABI85097 |
| CTL | M2 4-12 | LTEVETPIR (SEQ ID NO:14) | | ABD59884 |
| CTL | M2 4-13 | LTEVETPIRN (SEQ ID NO:15) | | ABD59884 |
| CTL | M2 6-14 | EVETPIRNE (SEQ ID NO:16) | | ABD59884 |
| CTL | M2 6-15 | EVETPIRNEW (SEQ ID NO:17) | | ABD59884 |
| CTL | M2 4-14 | LTEVETPIRNE (SEQ ID NO:18) | | ABD59884 |
| Th | M2 4-18 | | | ABD59884 |
| B cell | M2 6-13 | EVETPIRN (SEQ ID NO:20) | | ABD59900 |
| B cell | M2 1-18 | | | BAD89348 |
| B cell | M2 2-24 | | | BAD89348 |
| B cell | M2 2-24 | | | ABD59884 |
| B cell | M2 7-15 | VETPIRNEW (SEQ ID NO:24) | | ABD59884 |
| B cell | M1 2-12 | SLLTEVETYVL (SEQ ID NO:25) | | AAO52904 |
| CTL | M1 2-12 | SLLTEVETYVP (SEQ ID NO:26) | | AAO33507 |
| CTL | M1 3-11 | LLTEVETYV (SEQ ID NO:27) | | AAO33507 |
| CTL | M1 13-21 | SIVPSGPL (SEQ ID NO:28) | | ABD59901 |
| CTL | M1 17-31 | | | ABD59901 |
| CTL | M1 18-29 | GPLKAEIAQRLE (SEQ ID NO:30) | | ABD59901 |
| CTL | M1 27-35 | RLEDVFAGK (SEQ ID NO:31) | | ABD59901 |
| CTL | M1 41-51 | ALMEWLKTRPI (SEQ ID NO:32) | | ABD59901 |
| CTL | M150-59 | PILSPLTKGI (SEQ ID NO:33) | | ABD59901 |
| CTL | M1 51-59 | ILSPLTKGI (SEQ ID NO:34) | | ABD59901 |
| CTL | M1 55-73 | | | ABD59901 |
| CTL | M1 56-68 | TKGILGFVFTLTV (SEQ ID NO:36) | | ABD59901 |
| CTL | M1 57-68 | KGILGFVFTLTV (SEQ ID NO:37) | | ABD59901 |
| CTL | M1 58-66 | GILGFVFTL (SEQ ID NO:38) | | ABD59901 |
| CTL | M1 60-68 | LGFVFTLTV (SEQ ID NO:39) | | ABD59901 |
| CTL | M1 59-67 | ILGFVFTLT (SEQ ID NO:40) | | ABD59901 |
| CTL | M1 128-135 | ASCMGLIY (SEQ ID NO:41) | | ABD59901 |
| CTL | M1 134-142 | RMGAVTTEV (SEQ ID NO:42) | | ABD59901 |
| CTL | M1 145-155 | GLVCATCEQIA (SEQ ID NO:43) | | ABD59901 |
| CTL | M1 164-172 | QMVATTNPL (SEQ ID NO:44) | | ABD59901 |
| CTL | M1 164-173 | QMVATTNPLI (SEQ ID NO:45) | | ABD59901 |
| CTL | M1 178-187 | RMVLASTTAK (SEQ ID NO:46) | | ABD59901 |
| CTL | M1 232-240 | DLLENLQTY (SEQ ID NO:47) | | ABD59901 |

Nucleoprotein (NP) is one of the groups of specific antigens, which distinguishes between influenza A, B and C viruses. In contrast to HA, NP is highly conserved, being 94% conserved in all influenza A viruses. Influenza A virus NP-specific antibody has no virus neutralizing activity, but NP is an important target for cytotoxic T lymphocytes (CTL) which are cross-reactive with all type A viruses (Townsend, 1984). CTL recognize short synthetic peptides corresponding to linear regions of the influenza NP molecule.

Hemagglutinin (HA) is a glycoprotein trimer embedded in the influenza envelope. It has responsible for the attachment and penetration of the virus to the host cell. Antibodies to the HA neutralize viral infectivity. Antigenic variations of this molecule are responsible for frequent outbreaks of influenza and for the poor control of infection by immunization (Ada and Jones, 1986).

The influenza virus RNA polymerase is a heterocomplex composed of the three polymerase (P) proteins PB1, PB2 and PA-present in a 1:1:1 ratio. Their role in influenza virulence has not been fully elucidated. Non-limiting examples of HA, NP and PB peptide epitopes can be found in table 2 herein below.

**Table 2: HA, NP and PB peptide epitopes.**

| Epitope Type* | Epitope Position | Amino Acid Sequence | Nucleotide Sequence | NCBI accession |
|---|---|---|---|---|
| B cell | HA 91-108 | | | AAM82562 |
| B cell | HA 91-108 | | | GAC81017 |
| B cell | HA 107-124 | | | ABD59854 (from A/TW/3286/0 3 (H3N2) |
| B cell | HA 166-175 (HA 150-159 A/PR/8 strain) | WLTEKEGSYP (SEQ ID NO:50) | | ABD77675 |
| Th | HA 306-324 | | | AAL62329 |
| CTL | HA 521-531 | GVKLESMGIYQ (SEQ ID NO:53) | | ABJ09518 |
| CTL | HA 518-528 | EISGVKLESMG (SEQ ID NO:54) | | ABJ09518 |
| CTL | HA 458-467 | NVKNLYEKVK (SEQ ID NO:55) | | ABD77675 |
| Th | HA 128-145 | | | AAO46269 |
| Th | HA 307-319 (HA 306-318) | PKYVKQNTLKLAT (SEQ ID NO:57) | | AAL62329 |
| Th | NP 91-99 | KTGGPIYRR (SEQ ID NO:58) | | BAA99400 |
| CTL | NP 44-52 | CTELKLSDY (SEQ ID NO:59) | | BAA99400 |
| CTL | NP 82-95 | HPSAGKDPKKTGGP (SEQ ID NO:60) | | BAA99400 |
| CTL | NP 82-94 | HPSAGKDPKKTGG (SEQ ID NO:61) | | BAA99400 |
| Th | NP 206-229 | | | ABD59868 |
| CTL | NP 265-273 | ILRGSVAHK (SEQ ID NO:63) | | BAA99400 |
| CTL | NP 305-313 | KLLQNSQVY (SEQ ID NO:64) | | ABD59868 |
| CTL | NP 335-349 | | | ABD35694 |
| CTL | NP 335-350 | | | ABK34765 |
| CTL | NP 335-350 | | | ABD35694 |
| CTL | NP 380-393 | ELRSRYWAIRTRSG (SEQ ID NO:68) | | ABK34765 |
| CTL | NP 380-388 | ELRSRYWAI (SEQ ID NO:69) | | ABK34765 |
| CTL | NP 383-391 | SRYWAIRTR (SEQ ID NO:70) | | BAA99400 |
| CTL | NP 384-394 | YWAIRTRSGG (SEQ ID NO:71) | | BAA99400 |
| CTL | NP 382-390 | SRYWAIRTR (SEQ ID NO:72) | | BAA99400 |
| CTL | NP 418-426 | LPFDKPTIM (SEQ ID NO:73) | | BAA99400 |
| CTL | PB1 591-599 | VSDGGPNLY (SEQ ID NO:74) | | ABK34974 |
| CTL | PB1 571-579 | RRSFELKKL (SEQ ID NO:75) | | ABK34974 |
| CTL | PB2 368-376 | RRATAILRK (SEQ ID NO:76) | | ABK34762 |

In some examples the vaccine comprises a peptide epitope derived from influenza B. Non-limiting examples of influenza B peptide epitopes are shown in Table 3.

**Table 3: Influenza B peptide epitopes**

| Epitope type* | Epitope position | Amino acid sequence | Nucleotide sequence | NCBI accession |
|---|---|---|---|---|
| CTL | NP 30-38 (flu B) | RPIIRPATL (SEQ ID NO:77) | | ABF21293 |
| CTL | NP 263-271 (flu B) | ADRGLLRDI (SEQ ID NO:78) | | ABF21293 |
| Th | HA 308-320 (flu B) | PYYTGEHAKAIGN (SEQ ID NO:79) | | ABI84095 |
| B | HA 354-372 (flu B) | | | ABI83926 |

| | | | | |
|---|---|---|---|---|
| * Each peptide may belong to one or more epitope types. For example, a peptide that elicits a B-cell response can also elicit a T-cell (Th and/or CTL) response. | | | | |

### Nucleic Acids

Also disclosed are nucleic acid molecules encoding a vector such as an expression vector comprising the influenza epitopes and a host cell comprising a vector which comprises an influenza epitope useful in the preparation of a synthetic vaccine of the invention.

An isolated nucleic acid sequence encoding a peptide can be obtained from its natural source, for example as a portion of a gene. A nucleic acid molecule can also be produced using recombinant DNA technology (e.g., polymerase chain reaction (PCR) amplification, cloning) or chemical synthesis. Nucleic acid sequences include natural nucleic acid sequences and homologs thereof, comprising, but not limited to, natural allelic variants and modified nucleic acid sequences in which nucleotides have been inserted, deleted, substituted, and/or inverted in such a manner that such modifications do not substantially interfere with the nucleic acid molecule's ability to encode a functional flagellin.

A polynucleotide or oligonucleotide sequence can be deduced from the genetic code of a protein, however, the degeneracy of the code must be taken into account, and nucleic acid sequences also include sequences, which are degenerate as a result of the genetic code, which sequences may be readily determined by those of ordinary skill in the art.

The terms "nucleic acid" and "polynucleotide" as used herein refer to an oligonucleotide, polynucleotide or nucleotide and fragments or portions thereof, and to DNA or RNA of genomic or synthetic origin, which may be single- or double-stranded, and represent the sense or antisense strand. The term should also be understood to include, as equivalents, analogs of either RNA or DNA made from nucleotide analogs, and, as applicable to the embodiment being described.

The term "oligonucleotide" refers to a nucleic acid sequence of at least about 6 nucleotides to about 60 nucleotides, preferably about 15 to 30 nucleotides, and more preferably about 20 to 25 nucleotides, which can be used in PCR amplification or a hybridization- assay, or a microarray. As used herein, oligonucleotide is substantially equivalent to the terms "amplimers", "primers", "oligomers", and "probes", as commonly defined in the art. The oligonucleotides encoding the specific peptide epitopes useful in the preparation of the vaccine of the present invention are provided in tables 1-3 hereinabove.

As used herein, highly stringent conditions are those, which are tolerant of up to about 5% to about 25% sequence divergence, preferably up to about 5% to about 15%. Without limitation, examples of highly stringent (-10°C below the calculated Tm of the hybrid) conditions use a wash solution of 0.1 X SSC (standard saline citrate) and 0.5% SDS at the appropriate Ti (incubation temperature) below the calculated Tm of the hybrid. The ultimate stringency of the conditions is primarily due to the washing conditions, particularly if the hybridization conditions used are those, which allow less stable hybrids to form along with stable hybrids. The wash conditions at higher stringency then remove the less stable hybrids. A common hybridization condition that can be used with the highly stringent to moderately stringent wash conditions described above is hybridization in a solution of 6 X SSC (or 6 X SSPE), 5 X Denhardt's reagent, 0.5% SDS, 100 µg/ml denatured, fragmented salmon sperm DNA at an appropriate Ti. (See generally Sambrook et al., Molecular Cloning: A Laboratory Manual, 2d edition, Cold Spring Harbor Press (1989) for suitable high stringency conditions).

Stringency conditions are a function of the temperature used in the hybridization experiment and washes, the molarity of the monovalent cations in the hybridization solution and in the wash solution(s) and the percentage of formamide in the hybridization solution. In general, sensitivity by hybridization with a probe is affected by the amount and specific activity of the probe, the amount of the target nucleic acid, the detectability of the label, the rate of hybridization, and the duration of the hybridization. The hybridization rate is maximized at a Ti of about 20°C -25°C below Tm for DNA:DNA hybrids and about 10°C -15°C below Tm for DNA:RNA hybrids. It is also maximized by an ionic strength of about 1.5M Na⁺. The rate is directly proportional to duplex length and inversely proportional to the degree of mismatching.

Specificity in hybridization, however, is a function of the difference in stability between the desired hybrid and "background" hybrids. Hybrid stability is a function of duplex length, base composition, ionic strength, mismatching, and destabilizing agents (if any). The Tm of a perfect hybrid may be estimated for DNA:DNA hybrids using the equation of Meinkoth et al (1984).

### Chimeric or Recombinant Molecules

A "chimeric protein", "chimeric polypeptide" or "recombinant protein" are used interchangeably and refer to an influenza peptide epitope operatively linked to a polypeptide other than the polypeptide from which the peptide epitope was derived. The peptide epitopes of the present invention can be prepared by expression in an expression vector as a chimeric protein. The methods to produce a chimeric or recombinant protein comprising an influenza peptide epitope are known to those with skill in the art. A nucleic acid sequence encoding an influenza peptide epitope can be inserted into an expression vector for preparation of a polynucleotide construct for propagation and expression in host cells.

In a non-limiting example, the chimeric polypeptide disclosed includes chimeras of an influenza peptide epitope with one of the following polypeptides: flagellin, Cholera toxin, Tetanus toxin, Ovalbumin, Tuberculosis heat shock protein, Diphtheria Toxoid, Protein G from respiratory syncytial virus, Outer Membrane Protein from *Neisseria meningitides,* nucleoprotein (N) of vesicular stomatitis virus, glycoprotein (G) of vesicular stomatitis virus, Plasmodium falciparum Antigen Glutamate-Rich Protein, Merozoite Surface Protein 3 or Viruses envelope(E) protein.

The term "expression vector" and "recombinant expression vector" as used herein refers to a DNA molecule, for example a plasmid, flagellin or virus, containing a desired and appropriate nucleic acid sequences necessary for the expression of the recombinant peptide epitopes for expression in a particular host cell. As used herein "operably linked" refers to a functional linkage of at least two sequences. Operably linked includes linkage between a promoter and a second sequence, for example an nucleic acid of the present invention, wherein the promoter sequence initiates and mediates transcription of the DNA sequence corresponding to the second sequence.

The regulatory regions necessary for transcription of the peptide epitope can be provided by the expression vector. The precise nature of the regulatory regions needed for gene expression may vary among vectors and host cells. Generally, a promoter is required which is capable of binding RNA polymerase and promoting the transcription of an operably-associated nucleic acid sequence. Regulatory regions may include those 5' non-coding sequences involved with initiation of transcription and translation, such as the TATA box, capping sequence, CAAT sequence, and the like. The non-coding region 3' to the coding sequence may contain transcriptional termination regulatory sequences, such as terminators and polyadenylation sites. A translation initiation codon (ATG) may also be provided.

In order to clone the nucleic acid sequences into the cloning site of a vector, linkers or adapters providing the appropriate compatible restriction sites during synthesis of the nucleic acids. For examples, a desired restriction enzyme site can be introduced into a fragment of DNA by amplification of the DNA by use of PCR with primers containing the desired restriction enzyme site.

An expression construct comprising a peptide epitope sequence operably associated with regulatory regions can be directly introduced into appropriate host cells for expression and production of the peptide epitopes per se or as recombinant fusion proteins. The expression vectors that may be used include but are not limited to plasmids, cosmids, phage, phagemids, flagellin or modified viruses. Typically, such expression vectors comprise a functional origin of replication for propagation of the vector in an appropriate host cell, one or more restriction endonuclease sites for insertion of the desired gene sequence, and one or more selection markers.

The recombinant polynucleotide construct comprising the expression vector and a peptide epitope should then be transferred into a bacterial host cell where it can replicate and be expressed. This can be accomplished by methods known in the art. The expression vector is used with a compatible prokaryotic or eukaryotic host cell which may be derived from bacteria, yeast, insects, mammals and humans.

A particularly preferred expression vector is a flagellin vector. A non-limiting example of a flagellin expression vector is disclosed in US 6,130,082 incorporated herein by reference. Other expression vectors which include a flagella gene, for example a salmonella fliC gene are also suitable. The host cells which express the recombinant flagellin can be formulated as live vaccines.

An alternative to producing the peptide epitopes by recombinant techniques is peptide synthesis by use of a peptide synthesizer. Conventional peptide synthesis or other synthetic protocols well known in the art may be used.

### Vaccine Formulation

The vaccine can be formulated for administration in one of many different modes. According to one embodiment of the invention, the vaccine is administered intranasally. The intranasal composition can be formulated, for example, in liquid form as nose drops, spray, or suitable for inhalation, as powder, as cream, or as emulsion. The composition can contain a variety of additives, such as adjuvant, excipient, stabilizers, buffers, or preservatives. For straightforward application, the vaccine composition is preferably supplied in a vessel appropriate for distribution of the recombinant flagellin in the form of nose drops or an aerosol. In certain preferred embodiments the vaccine is formulated for mucosal deliver, in particular nasal delivery (Arnon, 2001; Ben-Yedidia, 1999).

In another embodiment of the invention, administration is oral and the vaccine may be presented, for example, in the form of a tablet or encased in a gelatin capsule or a microcapsule.

In yet another embodiment, the vaccine is formulated for parenteral administration. In some embodiments the vaccine is formulated for mass inoculation, for example for use with a jet-injector or a single use cartridge.

The formulation of these modalities is general knowledge to those with skill in the art.

The liposome provides another delivery system for antigen delivery and presentation. Liposomes are bilayered vesicles composed of phospholipids and other sterols surrounding a typically aqueous center where antigens or other products can be encapsulated. The liposome structure is highly versatile with many types range in nanometer to micrometer sizes, from about 25 nm to about 500 µm. Liposomes have been found to be effective in delivering therapeutic agents to dermal and mucosal surfaces. Liposomes can be further modified for targeted delivery by for example, incorporating specific antibodies into the surface membrane, or altered to encapsulate bacteria, viruses or parasites. The average survival time of the intact liposome structure can be extended with the inclusion of certain polymers, for example polyethylene glycol, allowing for prolonged *release in vivo.*

Microparticles and nanoparticles employ small biodegradable spheres which act as depots for vaccine delivery. The major advantage that polymer microspheres possess over other depot-effecting adjuvants is that they are extremely safe and have been approved by the Food and Drug Administration in the US for use in human medicine as suitable sutures and for use as a biodegradable drug delivery system (Langer, 1990). The rates of copolymer hydrolysis are very well characterized, which in turn allows for the manufacture of microparticles with sustained antigen release over prolonged periods of time (O'Hagen, et al., 1993).

Parenteral administration of microparticles elicits long-lasting immunity, especially if they incorporate prolonged release characteristics. The rate of release can be modulated by the mixture of polymers and their relative molecular weights, which will hydrolyze over varying periods of time. Without wishing to be bound to theory, the formulation of different sized particles (1 µm to 200 µm) may also contribute to long-lasting immunological responses since large particles must be broken down into smaller particles before being available for macrophage uptake. In this manner a single- injection vaccine could be developed by integrating various particle sizes, thereby prolonging antigen presentation and greatly benefiting livestock producers.

In some applications an adjuvant or excipient may be included in the vaccine formulation. The choice of the adjuvant will be determined in part by the mode of administration of the vaccine. For example, non-injected vaccination will lead to better overall compliance and lower overall costs. A preferred mode of administration is intranasal administration. Non-limiting examples of intranasal adjuvants include chitosan powder, PLA and PLG microspheres, QS-21, calcium phosphate nanoparticles (CAP) and mCTA/LTB (mutant cholera toxin E112K with pentameric B subunit of heat labile enterotoxin).

### Therapeutic Use of Vaccine

The vaccines of the invention are intended both for use in humans and in animals including livestock, poultry and domestic animals, for prevention or attenuation of influenza A and B disease.

The following examples are presented in order to more fully illustrate some embodiments of the invention. They should, in no way be construed, however, as limiting the broad scope of the invention. One skilled in the art can readily devise many variations and modifications of the principles disclosed herein without departing from the scope of the invention.

### EXAMPLES

ABBREVIATIONS: Ab: Antibodies; CTL: Cytotoxic T-lymphocytes; EID: Egg-infective dose; HA: Hemagglutinin; HAU: Hemagglutination unit; NP: Nucleoprotein; PMBC: Peripheral blood mononuclear cells; Th: T helper; IN or i.n.: intranasal; IP: intraperitoneal; IM or i.m.: intramuscular; NK: natural killer cells; E:T ratio: effector target ratio.

### EXAMPLE 1. Synthesis of peptides and recombinant flagellin

The epitopes may be expressed as recombinant flagella, wherein each flagella comprises one or more peptide epitopes. Alternatively, the peptide epitopes can be expressed in an expression vector as a multimeric fusion protein comprising two or more peptide epitopes.

The recombinant flagellin are synthesized by molecular biology methods known in the art. In brief: a flagella expression vector comprising an antibiotic marker was prepared. The synthesized oligonucleotides were inserted at the EcoRV site of the plasmid and transfected into E. *coli* competent cells. Colonies containing the recombinant plasmid were selected by probing with a radiolabeled oligonucleotide. Plasmids from positive colonies were purified and the insert orientation was determined using restriction analysis. The desired plasmids were used to transform *Salmonella typhimurium* LB5000 (a restrictive negative, modification proficient non flagellated) competent cells and were then transferred to a flagellin negative live vaccine strain (an Aro A mutant) of *Salmonella dublin* SL5928 by transduction using the phage P22HT105/1 int. The transformed *S*. *dublin* were selected for kanamycin resistance, motility under the light microscope and growth in semisolid LB agar plates, supplemented with Oxoid nutrient broth no. 2. Selected clones were grown overnight in 2 liters of medium and the flagellin purified by acidic cleavage. The resulting product is an aggregate of the flagellin and may be used as such for a vaccine.

The recombinant flagellin is not intended to be limited by the choice of the flagellin sequence. In some embodiments the flagellin having accession number X03395 Salmonella muenchen H1-d gene for phase-1-d flagellin (ATCC 8388).

### EXAMPLE 2: Response of chimeric mice to whole inactivated influenza virus

Human mouse chimeras are used to evaluate the immunogenic response elicited by the vaccine of the present invention. In order to establish the suitability of the human/mouse radiation chimera for evaluating the peptide-based vaccine, their immune response to inactive purified influenza virus was evaluated. The mice were immunized i. p. with 50 µg of the virus on the day of PBMC transplantation, followed by a sublethal viral challenge with influenza A/Texas/1/77 strain 14 days after immunization. The vaccination of human/mouse radiation chimera with the whole killed virus vaccine, without any adjuvant, induced production of specific antibodies-the serum antibody titer was significantly higher (2.4 fold) in the immunized chimera as compared to the control group. Moreover, this vaccination markedly reduced the subsequent virus infection. The lung virus titer after challenge was significantly lower (by 2.7 orders of magnitude) in the immunized chimera as compared to the control group.

This experiment demonstrates the suitability of the human/mouse radiation chimera for evaluating the anti-influenza response following immunization.

### EXAMPLE 3. FACS analysis of immunized mice for evaluating the engraftment of human PBMC in human/BALB chimera

The successful engraftment of the human cells in the human/mouse chimeras demonstrated in a preliminary experiment showing that most of the lymphocytes in the peritoneum (50-80%) and in the lungs of the mice (30-60%) were of human origin. For the evaluation of human cell engraftment in the human/mouse chimera, the presence of human cells in the engrafted mice was analyzed by FACS.

### EXAMPLE 4. Virus clearance from the lungs following sub-lethal challenge

Influenza infection is a respiratory disease; hence, a local immune response induced by an intranasal administration of the vaccine may be more efficient than parenteral administration. The immunization schedule was modified in order to adapt it for intranasal immunization.

Mice (6-8 per group in 7 repeated experiments) are immunized intranasally (i. n.) 10-12 days after PBMC transplantation, as described in the Methods. Ten days later, they are challenged i. n. with 10-4 HAU in 50µl allantoic fluid of live A/Texas/1/77 strain or another strain of influenza virus. Five days later they were sacrificed and their lungs were removed for virus titration. Human antibody production in these mice is evaluated in both the serum (before challenge) and in the lungs (after challenge).

Further to the sub-lethal infection challenge experiment, the ability of the vaccine to protect human/mouse chimera from a lethal dose of influenza virus is examined.

### EXAMPLE 5. Protection from infection with different strains of influenza

One of the major problems with currently available influenza vaccines is that they are effective only against the strains included in the vaccine. Therefore, it is of interest to examine the ability of the recombinant flagellin comprising influenza epitopes to protect mice from different influenza subtypes. The M peptide epitope, which is expressed in the flagellin, may be conserved in all influenza H3 subtypes, while the T-cell epitopes are from regions of the haemagglutinin and nucleoprotein highly conserved in other subtypes as well. In other examples the M peptideepitope is conserved in all H9 or H5 strains. In the first step, it is shown that rabbit antibodies towards these epitopes can indeed recognize and react in ELISA with different strains of influenza including A/Texas/1/77, A/Aichi/68, A/PR/8/34 and A/Japanese/57. To further test the potential of these epitopes to confer cross protection in humans, the human/mouse radiation chimera (8 mice per group) were immunized i. n. with the recombinant flagellin. Their resistance to different influenza strains challenge was detected 7 days later and compared to non-transplanted mice that are immunized with the same flagella mixture. The influenza strains used for infection were: A/Texas/1/77 (H3N2), A/Japanese/57 (H2N2) and A/PR/8/34 (H1N1). Other strains are tested, as well.

### EXAMPLE 6: CTL response assay

Following immunization with the vaccine of the present invention, the animals' spleens are removed and incubated with the peptide representing the relevant epitope (for example NP 335-350 or M2 1-18) for an additional 5 days. TAP deficient syngeneic target cells (with the same HLA A2.1 typing, for example: RMA cells) are incubated with the same peptide and with S³⁵-Methionine in a separate dish. The activated splenocytes are incubated with the target cell and upon recognition of the peptide, will attack them and release of methionine. High radioactivity counts indicate a high level of active CTL cells.

### EXAMPLE 7: Serum antibody and antibody neutralization assays

Mice, guinea pigs or rabbits are immunized with the mixture of recombinant flagellin, 14 days post immunization, blood samples are taken and serum is separated. These sera can be employed for detection the specificity of the antibodies, for example by ELISA or western blot, in addition, it is possible to determine their type, i.e. IgG1, or IgG2a or IgG2c etc. In lung homogenates IgA can be detected, indicative of a mucosal immune response.

Neutralization assay: MDCK cells are infected by influenza virus as determined by plaque counts or by MTT (sort of viability staining), following incubation of the virus with serum from immunized animals; the antibodies bind the virus and prevent infection of the MDCK cells. A reduced number of plaques or increased viability of the cells indicate the specificity of the antibodies and their ability to prevent/reduce infection.

### EXAMPLE 8: Immunogenicity of individual epitopes

HHD/HLA A2 transgenic mice were immunized with flagella expressing each epitope (Fla-91, Fla-335, Fla-380, Fla-M1 2-12) or with a mixture of 6 epitopes (Hexavaccine1: Fla-91, Fla-335, Fla-380, Fla-M1 2-12, Fla-354 and Fla-307). Fla-91 refers to flagella comprising HA 91-108 epitope; Fla-335 refers to flagella comprising NP 335-350 epitope; Fla-380 refers to flagella comprising NP 380-393 epitope; Fla-M1 2-12 refers to flagella expressing M1 2-12 epitope; Fla-354 refers to flagella expressing HA 354-372 (influenza B) epitope; Fla-307 refers to flagella expressing HA 307-319 epitope.

The cells were incubated with the respective peptide and the cells from Hexa-vaccine immunized mice were incubate with the 4 peptides HA91, NP335, NP380, M1 2-12. The peptide epitope sequences, sequence identifiers and strain homology data can be founds in table 4 herein below.

**Table 4: Hexa vaccine1 peptide epitope list**

| Epitope type | Epitope sequence | Homology in Influenza strains |
|---|---|---|
| Th | HA 307-319 PKYVKQNTLKLAT (SEQ ID NO:57) | H3N2 |
| CTL | NP 335-350 SAAFEDLRVLSFIRGY (SEQ ID NO:67) | H1N2, H2N2, H3N2, H9N2. |
| CTL | NP 380-393 ELRSRYWAIRTRSG (SEQ ID NO:68) | H1N1, H1N2, H2N2, H3N8, H5N1, H5N2, H5N9, H6N1, H6N2, H6N9, H7N7, H9N2, H9N2, H11N1, H11N8, H11N9, H14N5. |
| B-cell | HA 91-108 SKAYSNCYPYDVPDYASL (SEQ ID NO:48) | H3N2 |
| B-cell and CTL | M1 2-12 SLLTEVETYVP (SEQ ID NO:26) | H1N1, H3N2, H4N6, H5N1, H5N2, H5N3, H6N1, H7N3, H9N2, |
| B-cell (Influen za B) | HA 354-372 PAKLLKERGFFGAIAGFLE (SEQ ID NO:80) | B/HongKong/330/2001; B/Beijing/1/87; B/Singapore/222/79; B/Oregon/5/80; B/Shangdong/7/97; B/Memphis/13/03; B/Los Angeles/1/02; B/Nebraska/1/01; B/Hong Kong/548/2000; B/Hong Kong/156/99; B/Vienna/1/99; B/Lee/40 and others |

### Immunogenicity in "HHD Transgenic Mouse" Model

The CTL epitopes were selected for their binding to HLA-A2 molecules. In order to study HLA-restricted responses, the D b-/- β2 microglobulin (β2m) null mice, transgenic for a recombinant HLA-A2.1/D b-/-. β2 microglobulin single chain (HHD mice) was employed. These mice combine classical HLA transgenesis with selective destruction of murine H-2 and show only HLA-A2.1-restricted responses. These mice serve as an animal model for research of systems involving cellular immunity such as cancer, autoimmunity and vaccination issues.

The cellular response that contributes to the elimination of the virus involves cytokine-mediated mechanisms. The involvement of cytokines in the immune response mounted by the recombinant vaccine was studied in the HHD transgenic mice model.

In this study, flagella expressing various peptide epitopes in PBS, emulsified in Freund adjuvant, were administered subcutaneously three times. The control group was administered with PBS emulsified in Freund adjuvant.

Cellular assays: splenocytes of these mice were incubated in the presence of the synthetic peptides corresponding to the above epitope. IFN-y secretion by the cells in response to the stimulation with the peptides was monitored by ELISA.

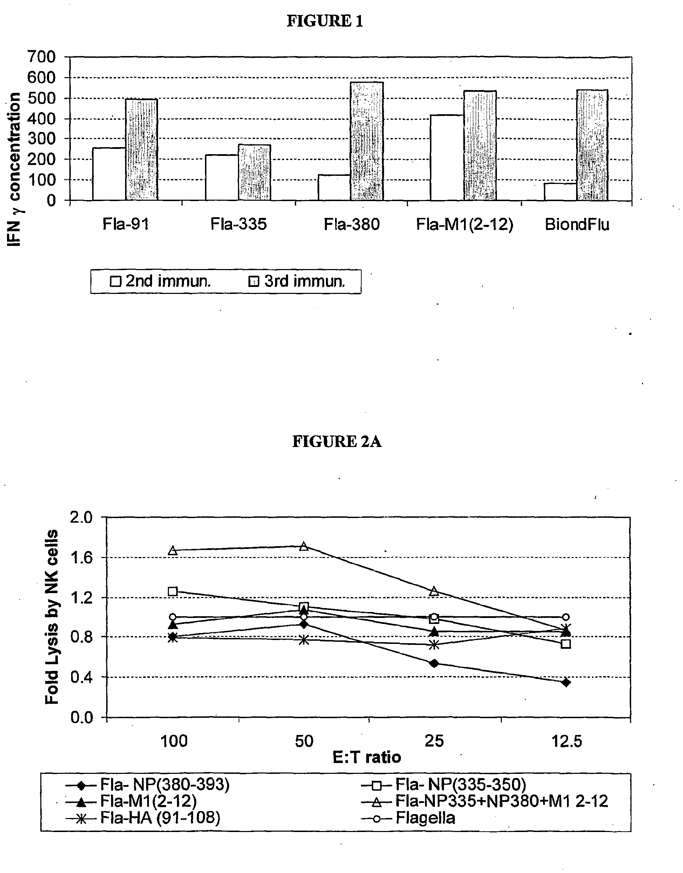

Figure 1 shows the IFN gamma secretion as measured from lymphocytes incubated with the respective peptide after the second and third immunization.

Activated lymphocytes secreted IFN-γ in response to incubation with the corresponding peptides. The group immunized with Hexa-vaccine1, containing the mixture of 6 recombinant flagella was incubated with a mixture of the 4 cellular epitopes tested separately. After the third immunization, The IFN-y secretion from these cells is significantly elevated (IFN levels secreted by cells incubated with medium, were below the assay detection level of 2pg/ml). The IFN gamma secreted from non activated lymphocytes (negative controls - grown in medium without peptide) was <0.004ng/ml.

NK (Natural Killer cell) lysis contributes to the anti viral response. It is known that viral infected cells are more sensitive to lysis than non-infected cells. It is speculated that the recognition of target cells by NK cells is more 'specific' than previously thought. A similarity in peptide motif between HLA A2 binders and HLA-G (expressed on NK cells) binders has been demonstrated.

Therefore, in addition to the non-specific mechanism of NK activation, peptides specific to HLA-A2 can elicit further specific elevation of NK activity (lysis).

Cytotoxic T lymphocyte (CTL) assays: HHD/HLA A2.1 mice were immunized with Hexa-vaccine1 in PBS. Direct lysis of target cells by CD8+ lymphocytes was not demonstrated, however, a marked lysis of Yac-1 cells that are sensitive to NK lysis was obtained. Lysis by NK was found after the second immunization and was further elevated after the third immunization. It should be noted that baseline levels of NK activation in naïve mice is approximately null.

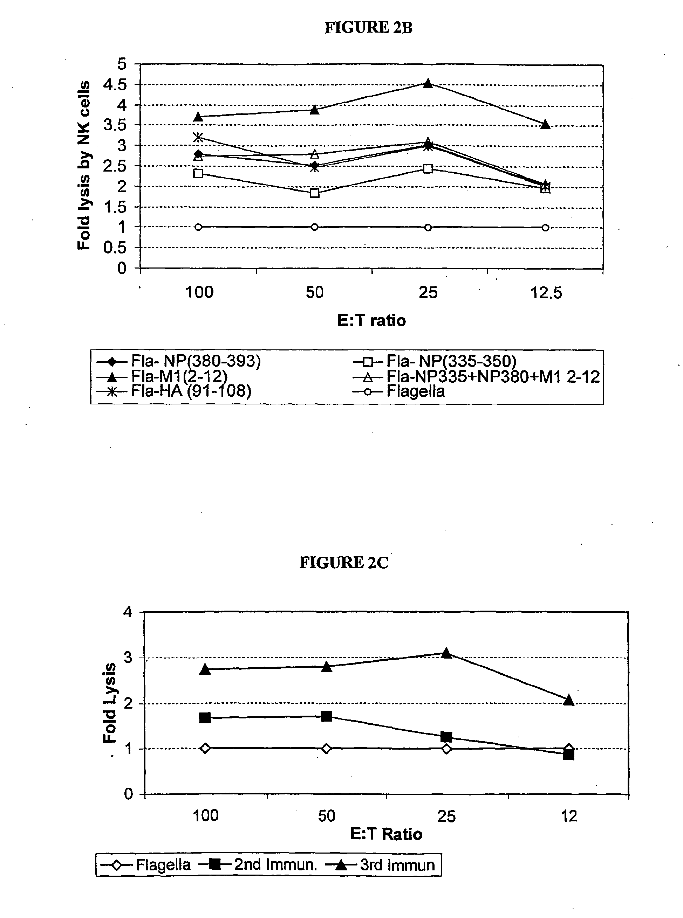

Figures 2A -2C shows lysis of target cells by NK derived from vaccinated mice was followed after the second and third immunization. Percentage of lysis of YAC-1 targets by NK cells after the second and third immunization is presented. Splenic lymphocytes from immunized mice were sensitized with the peptides included in the recombinant flagella for 5 days and then incubated with ³⁵S-Met labeled YAC-1 cells. Specific lysis was determined at different E:T ratios. The data (% lysis by NK cells of each group) is presented as fold activation in comparison to the lysis of the group immunized with native flagella. After the second immunization, NK cells from the mice immunized with the combination of 6 epitopes (Hexa-vaccine 1) were able to lyse the target cells more efficiently than the cells from mice vaccinated with the native flagella. Figures 2A and 2B show the NK cell lysis of individual recombinant peptide epitopes or a combination of three recombinant epitopes (Fla-NP335, Fla-NP380, Fla-Ml 2-12).

After three immunizations, the cells from mice immunized with M1 2-12 showed a significant elevation in their ability to destroy target cells. The M1 2-12 peptide epitope is therefore a useful peptide epitope in the preparation of the vaccine of the present invention.

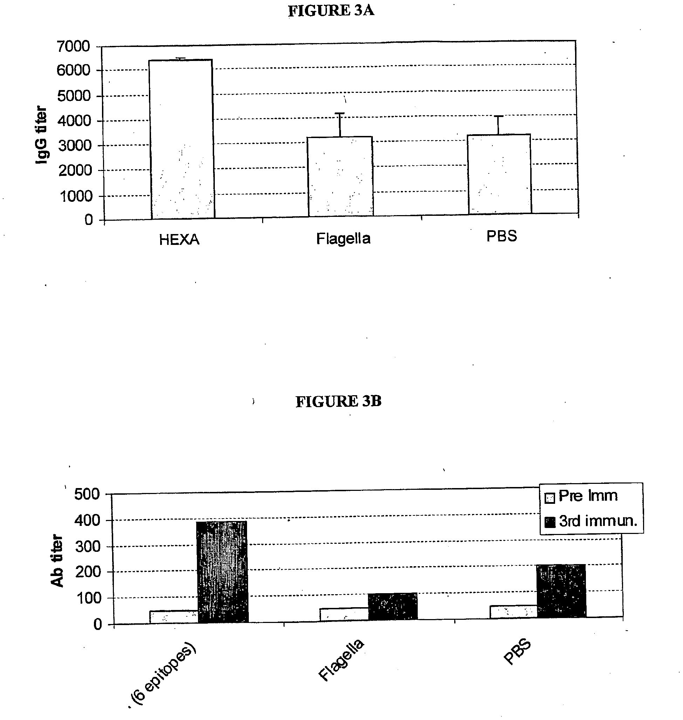

Figures 3A and 3B show the results of immunization of C57B1/6 mice with the Hexa-vaccine1, consisting of flagella with 6 influenza epitopes (HA 91-108, HA 354-372, HA 307-319, NP 335-350, NP 380-393 and M2 1-18):

Serum was removed after a schedule of 3 immunizations and the specificity of Ab against the whole H3N2 influenza virus (Figure 3A) and against the specific M2 1-18 peptide (Figure 3B) was determined.

Binding of epitopes and stabilization of HLA-A2 on human T2 (deficient for TAP transporters and therefore express low and unstable amounts of HLA-A2.1 molecules. Upon binding of peptides, stable and high levels of HLA-A2 are expressed on the cell surface). T2 cells were incubated with various concentrations of peptides in serum-free medium over night and stained with specific monoclonal antibodies. Stabilization of HLA-A2 molecules was detected by Flow cytometry.

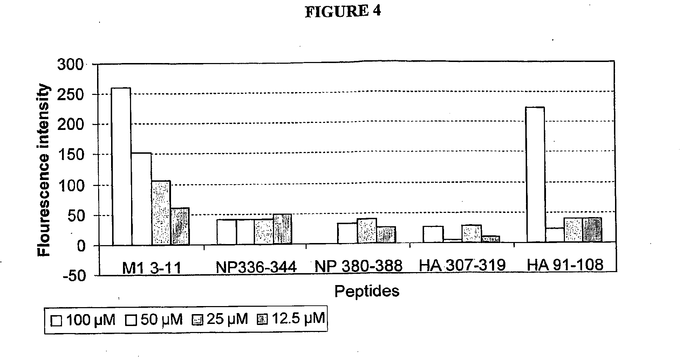

Figure 4 shows binding of peptides to HLA-A2 on T2 cells: High and dose dependent binding was shown by the M1 3-11 peptide. Other tested peptides, NP 336-344, NP 380-388 and HA 307-319, showed some low binding capacity, which was not dose dependent. NP 380-393 is known as specific to HLA B8 molecule and is not expected to bind HLA A2. The HA 91-108 peptide which is longer than the HLA groove, showed binding capacity only at the higher (100µM) concentration, probably due to the HLA motif in its N-terminal side. Minimal and not dose dependent binding was demonstrated at lower concentrations.

In addition to the cellular responses, sera from mice after the third immunization were compared to pre immune sera for antibodies specific to the epitopes:

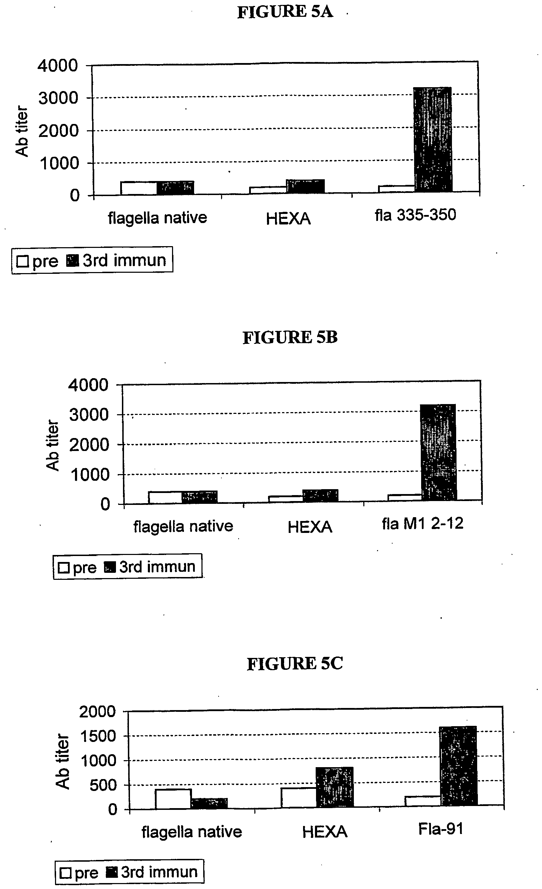

Figures 5A-5C shows that the recombinant peptide epitopes elicit antibody protection. A significant elevation in Ab titer, specific to the epitope was obtained with epitopes NP 335-350 (5A), M1 2-12 (5B) and HA 91-108 (5C) in mice immunized with the single relevant epitope (200µg/mouse, IM).

### Conclusions

The results of this study indicate that immunization with the recombinant flagellin containing specific T cells epitopes resulted in specific recognition and cellular TH1 type response, as shown by IFN-y secretion by lymphocytes from the vaccinated mice in response to in-vitro stimulation with the respective synthetic peptides. Furthermore, the specific binding of the investigated epitopes to HLA A2 expressing target cells as well as specific lysis of these cells loaded with the epitope by NK cells indicates cellular response to the Hexa-vaccine1 in HHD transgenic mice.

### EXAMPLE 9: Dose Optimization Study

This study was designed to select the optimal dose of the epitope based anti influenza vaccine Hexa-vaccine, which is the lowest effective dose conferring sufficient immune response as measured by various immunological tests. Performing this study with Hexa-vaccine assessed the efficacy of the vaccine by assessment of humoral response and virus titration in the lungs after a schedule of 3 immunizations and infection.

### Study Design

HHD/HLA A2.1 mice were immunized 3 times IN and IM with 240, 80, 24 or 8µg of Hexa-vaccine1, consisting of influenza epitopes within bacterial flagella in PBS (see Table 5 for groups and identification). Blood was collected for evaluation of the humoral response elicited in the vaccinated mice. The mice were infected with H3N2 influenza virus and viral titration in their lungs served as a correlate for efficacy.

**Table 5: Study Groups and Identifications**

| Group No. | Treatment | Route |
|---|---|---|
| A | 8 µg | IN |
| B | 24 µg | IN |
| C | 80 µg | IN |
| D | 240 µg | IN |
| E | flagella (Control) 240 µg | IN |
| F | 8 µg | IM |
| G | 24 µg | IM |
| H | 80 µg | IM |
| I | 240 µg | IM |
| J | Flagella (Control) 240 µg | IM |

### Humoral Immune response

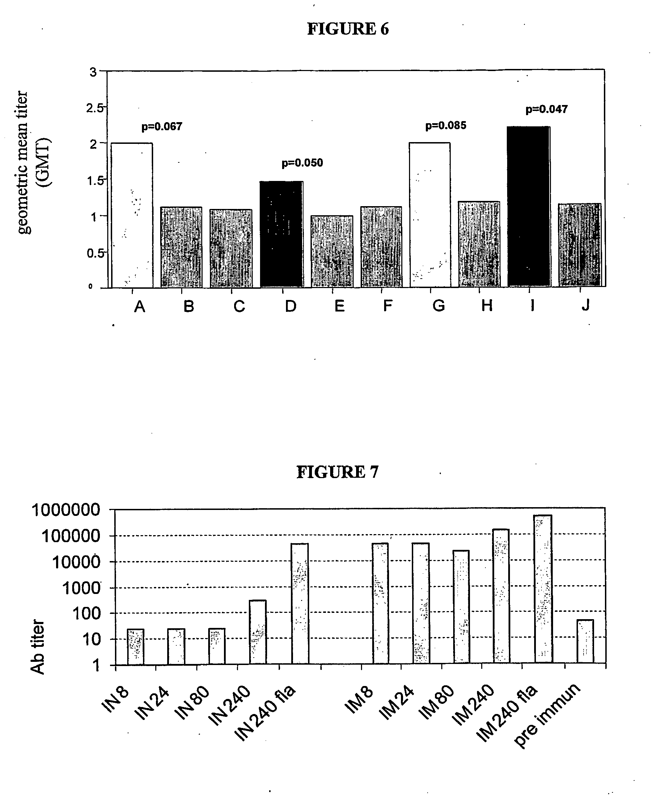

Escalating doses of Hexa-vaccine1 containing 8-240µg of all the epitopes combined induced a specific humoral response to the H3N2 virus. The response in the groups immunized with the higher dose was significantly higher than the baseline. The change of titer between pre immune and immunized sera is shown in Figure 6, which corresponds to the groups in Table 5. A significant elevation over the pre-immune level (p<0.05) and over the control groups E and J that were immunized with native flagella (IN or IM respectively in terms of specific recognition of influenza virus H3N2 is observed in groups D and I that were immunized with 240µg of Hexa-vaccine1 IN or IM, respectively.

### Humoral response: specific recognition of flagella

The humoral response is also directed towards the flagella carrier. The sera titer antibody to it is demonstrated in Figure 7

Figure 7 shows the humoral response to flagella following IN or IM administration of Hexa-vaccine1. In the IN immunized groups (left hand bars); there was an escalating dose response where the higher Ab production was found where the higher dose (IN 240µg) was given. In the IM administrated groups (right hand bars) a similarly high response was observed in all the doses ranging from 8µg -240µg/mouse. By both routes, the response to the native flagella is much higher; this may result from structural changes caused by the addition of foreign epitopes that may influence its immunogenicity.

### Virus titration

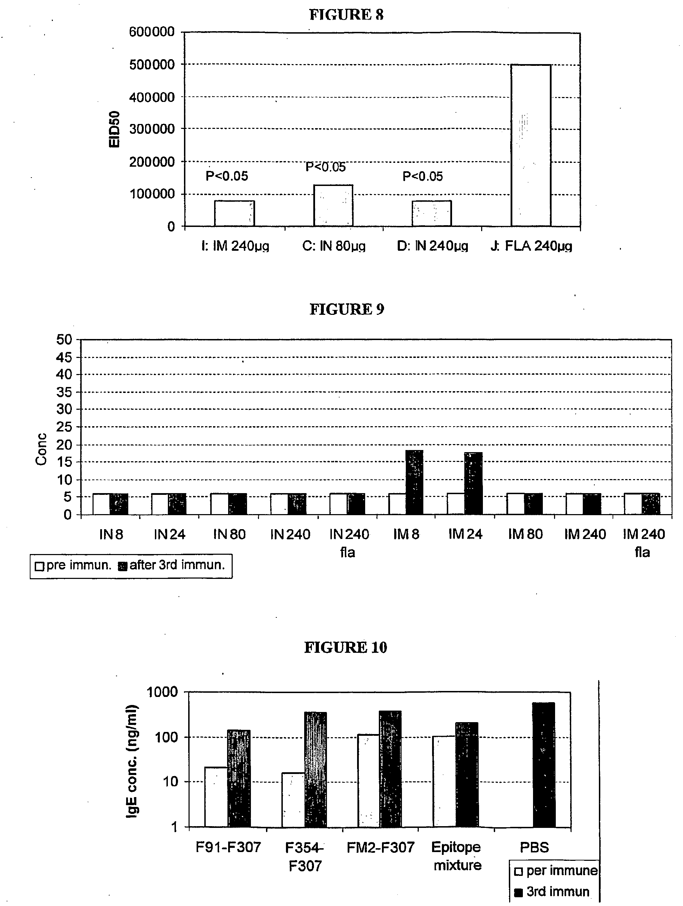

After a schedule of 3 vaccinations, the mice were infected with a sub-lethal dose of influenza virus H3N2 strain (A/Texas/1/77). The lungs of the mice were removed 5 days later for titration of viral load in them. The titration was performed in fertilized eggs figure 8). The viral load in the groups immunized with Hexa-vaccine1 (240µg) is comparable in both IM and IN routes and is significantly lower (p<0.05) than the titer in the control groups. This shows that Hexa-vaccine1 is effective and provides protection against virus infection.

### Conclusions

Antibodies to (H3N2) virus: were significantly higher in the group immunized with the higher doses both IM and IN, but not in the groups immunized with lower doses, showing a dose escalating response.

Virus clearance: a lower virus titer was found in the groups immunized IN and IM, while higher viral titers were detected in animals immunized with lower doses.

These data indicates that there is a correlation between dose and response, where only the higher doses led to significant protection.

No significant weight loss, behavioral abnormalities or signs for allergy (IgE elevation) were noticed as described in the following;

### EXAMPLE 10:IgE Response following Immunization

For the evaluation of potential allergic response to our product, IgE levels were measured in all major experiments conducted in mice following immunization with different combination of epitopes or with native flagella. In the various studies, mice were immunized IN or IM with the recombinant flagella, total IgE in the sera was measured by a commercial murine IgE detection kit.

Low titers of <20ng/ml were found in immunized animals similar to the normal level in non immunized mice.

Figure 9 presents the IgE concentration (ng/ml) in the dosing experiment and in the experiment for evaluating the cellular response. In both, IgE titers on day 0 and after the third immunization were similar.

Mice were immunized either IN or IM with Hexa-vaccine1 at escalating doses, from 8µg-240µg/mouse. The sera from these mice was tested for total IgE

Figure 10 represents the IgE concentration in sera of HHD transgenic (TG) mice immunized IN with recombinant flagella expressing influenza epitopes.

### Conclusions

In the sera samples from mice immunized with recombinant flagella, low levels of IgE were detected. The normal range for plasma IgE is established between 19 and 200 ng/ml using wild type animals). Titers obtained in immunized animal did not exceed 20ng/ml and therefore, Hexa-vaccine does not induce allergic responses.

### EXAMPLE 11: Evaluation of Hexa-vaccine2 in Rabbits

In this study rabbits were administered IN and IM with the vaccine product in a controlled environment at a Specific Pathogen Free (SPF) certified animal house. After a schedule of 3 vaccinations, blood samples were removed and specimens from the administration site and major organs were removed and subjected to histopathological analysis.

The study groups consisted of 3 immunizations comparing Hexa-vaccine2 (6 epitopes: HA 91-108, HA 307-319, HA 354-372, NP 335-350, NP 380-393, M1 2-12) to PBS:
IN route: Hexa-vaccine2 100 µg and 50µg/50µl/rabbit
IM route: Hexa-vaccine2 600 µg and 300µg/500µl/rabbit

Neither mortality nor morbidity was observed in any of the groups. Histopathology results (2 weeks post immunization) showed:
Intranasal: No toxicity in the organs examined.
Intramuscular: No toxicity in the organs examined. One animal presented focal, minimal histolytic infiltrate at injection site.

### Conclusions

Safety: The Hexa-vaccine2 was found to be safe and tolerable in rabbits. Humoral response: Ab titer specific to different influenza strains was recorded in some of the rabbits. It should be noted that distinct responses were found between individual rabbits. Figure 10 describes the fold increase in the antibody titer as compared to the pre immune titer in the responding rabbits. Non responding rabbits were excluded.

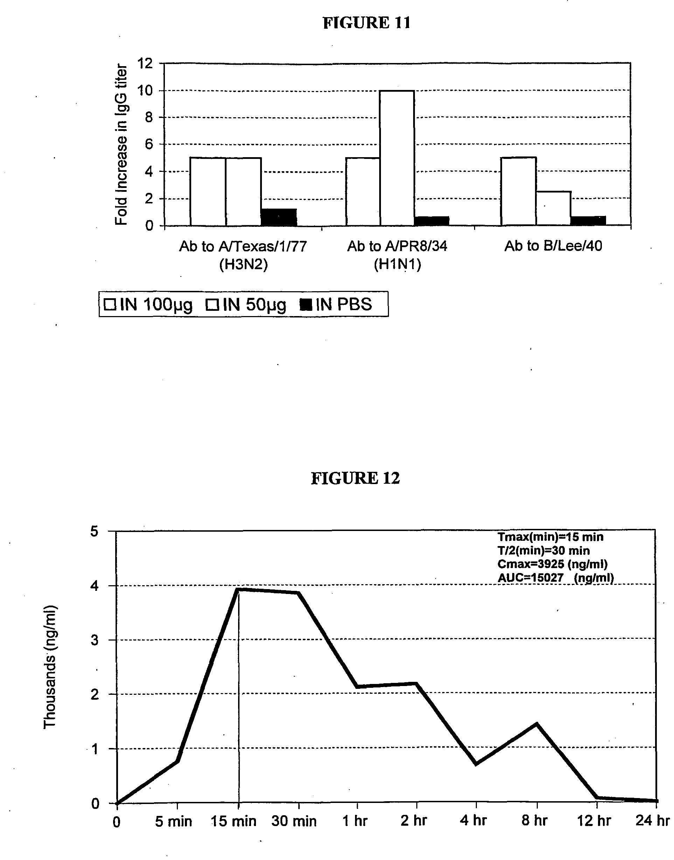

Figure 11: Fold IgG titer to 3 different influenza strains in sera of NZW rabbits immunized IN 3 times with recombinant flagella expressing 6 influenza epitopes.

### EXAMPLE 12: Pharmacokinetics

The pharmacokinetic studies revealed that the vaccine peak in sera was obtained at 15 minutes and eliminated within 12 hours. In a parallel study with the vaccine formulated with adjuvant (Alum), the peak concentration in sera was reached after 30 minutes and the vaccine was eliminated from the sera after 24 hours (data not shown). In addition, Cₘₐₓ, Tₘₐₓ and AUC values were calculated as described hereunder.

### Study design

The study consisted of 10 groups of 3 males and 3 females per group that were administered with a single dose of 50 µg recombinant flagella/animal. Animals were bled at 10 predetermined time points of 5, 10, 30 minutes and 1, 2, 4, 8, 12, 24 hours.

### Pharmacokinetics Analysis

Calculation of the pharmacokinetic characteristics were based on the actual blood sampling time [h] (relative to the corresponding administration time of Treatment) rounded to two decimal digits and negative pre dose times set to zero. The sample before administration was used for calculation of the characteristics.

For calculation of the pharmacokinetic parameters, the following rules were applied:

Flagellin concentration values in sera at time points in the lag-time between time zero and the first quantifiable concentration were considered as zero. Evaluation of relative bioavailability was performed for the primary target parameters AUC and Cₘₐₓ.

The log transformed values of the primary target parameters were subject to an analysis of variance (ANOVA) model with the effects: sequence, subjects within sequence, period and treatment. The sequence effect was tested using the mean square of subjects within sequence from the ANOVA as an error term. All other effects were tested against the residual error (error mean square) from the ANOVA. Based on the ANOVA 90% confidence intervals for the treatment ratios test* 100/reference [%] was calculated.

Individual treatment ratios test* 100/reference [%] was given for the primary target parameters. For Tₘₐₓ frequency tables were drawn by treatment based on the nominal time of the Tₘₐₓ values.

Figure 12 depicts protein serum concentration. Maximum serum concentration 3,925ng/ml (Cₘₐₓ) of Hexa-vaccine was observed after 15 minutes (Tₘₐₓ). Half (T_{½}) of the total exposure quantity was obtained within 30 minutes post dosing. The area under the serum concentration-time curve of 15,027ng/ml indicates the body's total exposure over time to Hexa-vaccine. No traces of protein could be detected after 12 h.

### Conclusions

A typical concentration curve was obtained at the end of the pharmacokinetics study with steep rise of the curve between 5 to 15 minutes and moderate slope up until 12 hours. The maximum concentration level (Cₘₐₓ = 3,925ng/ml) was observed upon 15 minutes, No traces of protein in the serum could be detected upon 12 hours post dosing. The flagellin based vaccine will be totally eliminated from the sera within 12 hours.

### Epitope Safety

The selected conserved epitopes utilized in the Hexa-vaccine comprise epitopes that are restricted to the most prevalent HLA molecules in human. The selected epitopes are restricted to the viral structure and are not shared by any human protein therefore they are unlikely to induce an autoimmune reaction.

### REFERENCES

Ada GL and Jones PD, "The immune response to influenza infection", Curr. Topics Microbio. Immunol.1986; 128:1.
Arnon R, Tarrab-Hazdai R, Ben-Yedidia T. Peptide-based synthetic recombinant vaccines with anti-viral efficacy. Biologicals. 2001; 29(3-4):237-42.
Ben-Yedidia T, Marcus H, Reisner Y, Arnon R. Intranasal administration of peptide vaccine protects human/mouse radiation chimera from influenza infection. Int Immunol. 1999; 11(7):1043-51.
Gianfrani C, Oseroff C, Sidney J, Chesnut R, Sette A. Human memory CTL response specific for influenza A virus is broad and multispecific. Hum Immunol. 2000; 61:438-452.
Ibrahim GF, Fleet GH, Lyons MJ, Walker RA. Method for the isolation of highly purified Salmonella flagellins. J Clin Microbiol. 1985; (6):1040-4.
Jeon SH, Ben-Yedidia T, Arnon R. Intranasal immunization with synthetic recombinant vaccine containing multiple epitopes of influenza virus. Vaccine. 2002; 20(21-22):2772-80.
Lamb R A, Zebedee S L, Richardson C D. Influenza virus M2 protein is an integral membrane protein expressed on the infected-cell surface. Cell. 1985; 40:627-633.
Langer R. New methods of drug delivery. Science. 1990; 249(4976):1527-33.
Liu W, Zou P, Ding J, Lu Y, Chen YH. Sequence comparison between the extracellular domain of M2 protein human and avian influenza A virus provides new information for bivalent influenza vaccine design. Microbes Infect. 2005; 7(2):171-7.
Meinkoth J, Wahl G. Hybridization of nucleic acids immobilized on solid supports. Anal Biochem. 1984;138(2):267-84.
O'Hagan DT, Jeffery H, Davis SS. Long-term antibody responses in mice following subcutaneous immunization with ovalbumin entrapped in biodegradable microparticles. Vaccine. 1993;11(9):965-9.
Shapira M, Jolivet M, Arnon R. A synthetic vaccine against influenza with built-in adjuvanticity. Int J Immunopharmacol. 1985;7:719-723.
Slepushkin VA, Katz JM, Black RA, Gamble WC, Rota PA, Cox NJ. Protection of mice against influenza A virus challenge by vaccination with baculovirus-expressed M2 protein. Vaccine. 1995; 13(15):1399-402.
Townsend AR, Skehel JJ. The influenza A virus nucleoprotein gene controls the induction of both subtype specific and cross-reactive cytotoxic T cells. J Exp Med. 1984; 160(2):552-63.
Zou P, Liu W, Chen YH: The epitope recognized by a monoclonal antibody in influenza A virus M2 protein is immunogenic and confers immune protection. Int Immunopharmacol. 2005; 5(4):631-5.

## Claims

1. A vaccine comprising the influenza virus peptide epitopes: M1 2-12 (SEQ ID NO:25 or 26); HA 91-108 (SEQ ID NO:48); HA 307-319 (SEQ ID NO:57); NP 335-350 (SEQ ID NO:67); NP 380-393 (SEQ ID NO:68); and HA 354-372 (SEQ ID NO:80).

2. The vaccine according to claim 1, further comprising an adjuvant or an excipient.

3. The vaccine according to claim 1, formulated for administration by a modality selected from the group consisting of intraperitoneal, subcutaneous, intranasal, intramuscular, oral, topical and transdermal delivery.

4. A vaccine according to any of the preceding claims for use in a method for eliciting an immune response and conferring protection against influenza virus in a subject.

5. The vaccine according to claim 4, wherein the immune response is elicited against avian influenza, influenza type A, influenza type B or a combination thereof.

6. Use of a vaccine according to any of the claims 1-5 for the preparation of a pharmaceutical agent for eliciting an immune response and conferring protection against influenza virus in a subject.

## Patentansprüche

1. Vakzin, welches die Influenzavirus-Peptid-Epitope umfasst: M1 2-12 (SEQ.ID.NR. 25 oder 26); HA 91-108 (SEQ.ID.NR. 48), HA 307-319 (SEQ.ID.NR. 57), NP 335-350 (SEQ.ID.NR. 67); NP 380-393 (SEQ.ID.NR. 68) und HA 354-372 (SEQ.ID:NR. 80).

2. Vakzin, nach Anspruch 1, welches weiter ein Adjuvant oder einen Exzipienten umfasst.

3. Vakzin nach Anspruch 1, das zur Verabreichung über eine Modalität formuliert ist ausgewählt aus der Gruppe bestehend aus intraperitonealer, subkutaner, intranasaler, intramuskulärer, oraler, topikaler und transdermaler Lieferung.

4. Vakzin nach einem der vorhergehenden Ansprüche zur Verwendung in einem Verfahren zum Auslösen einer Immunantwort und Verleihen von Schutz gegen Influenzavirus in einem Individuum.

5. Vakzin nach Anspruch 4, wobei die Immunantwort gegen Affen-Influenza, Influenza Typ A, Influenza Typ B oder eine Kombination davon hervorgerufen wird.

6. Verwendung eines Vakzins nach einem der Ansprüche 1-5 zur Herstellung eines pharmazeutischen Mittels zum Hervorrufen einer Immunantwort und Verleihen von Schutz gegen Influenzavirus in einem Individuum.

## Revendications

1. Vaccin comprenant les épitopes de peptides du virus de la grippe: M1 2-12 (SEQ ID NO:25 ou 26); HA 91-108 (SEQ ID NO:48); HA 307-319 (SEQ ID NO:57); NP 335-350 (SEQ ID NO:67); NP 380-393 (SEQ ID NO:68); et HA 354-372 (SEQ ID NO:80).

2. Vaccin selon la revendication 1, comprenant en outre un adjuvant ou un excipient.

3. Vaccin selon la revendication 1, formulé pour administration suivant une modalité sélectionnée parmi le groupe suivant: intrapéritonéale sous-cutanée, intranasale, intramusculaire, orale, locale ou transdermique.

4. Vaccin selon l'une quelconque des revendications précédentes pour utilisation dans un procédé pour susciter une réponse immunitaire et conférer une protection contre le virus de la grippe chez un sujet.

5. Vaccin selon la revendication 4, dans lequel la réponse immunitaire est sollicitée contre la grippe aviaire, la grippe de type A, la grippe de type B ou un combinaison de celles-ci.

6. Utilisation d'un vaccin selon l'une quelconque des revendications 1 à 5 pour la préparation d'un agent pharmaceutique pour susciter une réponse immunitaire et conférer une protection contre le virus de la grippe chez un sujet.


### SEQUENCE LISTING

<110> Yeda Research and Develoment Co. Ltd. at the Weizmann Institute of Science BEN-YEDIDIA, Tamar ARNON, Ruth
<120> IMPROVED INFLUENZA VACCINE
<130> BNDVX/001 PCT
<140> PCT/IL2006/
   <141> 2006-12-06
<150> 60/742,574
   <151> 2005-12-06
<150> 60/742,530
   <151> 2005-12-06
<160> 162
<170> PatentIn version 3.3
<210> 1
   <211> 4
   <212> PRT
   <213> INFLUENZA
<400> 1
   Glu Val Glu Thr
   1
<210> 2
   <211> 15
   <212> PRT
   <213> INFLUENZA
<400> 2
   Met Ser Leu Leu Thr Glu Val Glu Thr His Thr Arg Asn Gly Trp
   1 5 10 15
<210> 3
   <211> 9
   <212> PRT
   <213> INFLUENZA
<400> 3
   Pro Ile Arg Asn Glu Trp Gly Cys Arg
   1 5
<210> 4
   <211> 10
   <212> PRT
   <213> INFLUENZA
<400> 4
   Glu Thr Pro Ile Arg Asn Glu Trp Gly Cys
   1 5 10
<210> 5
   <211> 11
   <212> PRT
   <213> INFLUENZA
<400> 5
   Pro Ile Arg Asn Glu Trp Gly Cys Arg Cys Asn
   1 5 10
<210> 6
   <211> 9
   <212> PRT
   <213> INFLUENZA
<400> 6
   Leu Leu Thr Glu Val Glu Thr Pro Ile
   1 5
<210>. 7
   <211> 9
   <212> PRT
   <213> INFLUENZA
<400> 7
   Ser Leu Leu Thr Glu Val Glu Thr Pro
   1 5
<210> 8
   <211> 10
   <212> PRT
   <213> INFLUENZA
<400> 8
   Ser Leu Leu Thr Glu Val Glu Thr Pro Ile
   1 5 10
<210> 9
   <211> 9
   <212> PRT
   <213> INFLUENZA
<400> 9
   Leu Thr Glu Val Glu Thr Pro Leu Thr
   1 5
<210> 10
   <211> 15
   <212> PRT
   <213> INFLUENZA
<400> 10
   Met Ser Leu Leu Thr Glu Val Glu Thr Pro Ile Arg Asn Glu Trp
   1 5 10 15
<210> 11
   <211> 18
   <212> PRT
   <213> INFLUENZA
<400> 11
   Met Ser Leu Leu Thr Glu Val Glu Thr Pro Ile Arg Asn Glu Trp Gly
   1 5 10 15
   Cys Arg
<210> 12
   <211> 15
   <212> PRT
   <213> INFLUENZA
<400> 12
   Met Ser Leu Leu Thr Glu Val Glu Thr Leu Thr Lys Asn Gly Trp
   1 5 10 15
<210> 13
   <211> 15
   <212> PRT
   <213> INFLUENZA
<400> 13
   Met Ser Leu Leu Thr Glu Val Glu Thr Leu Thr Arg Asn Gly Trp
   1 5 10 15
<210> 14
   <211> 9
   <212> PRT
   <213> INFLUENZA
<400> 14
   Leu Thr Glu Val Glu Thr Pro Ile Arg
   1 5
<210> 15
   <211> 10
   <212> PRT
   <213> INFLUENZA
<400> 15
   Leu Thr Glu Val Glu Thr Pro Ile Arg Asn
   1 5 10
<210> 16
   <211> 9
   <212> PRT
   <213> INFLUENZA
<400> 16
   Glu Val Glu Thr Pro Ile Arg Asn Glu
   1 5
<210> 17
   <211> 10
   <212> PRT
   <213> INFLUENZA
<400> 17
   Glu Val Glu Thr Pro Ile Arg Asn Glu Trp
   1 5 10
<210> 18
   <211> 11
   <212> PRT
   <213> INFLUENZA
<400> 18
   Leu Thr Glu Val Glu Thr Pro Ile Arg Asn Glu
   1 5 10
<210> 19
   <211> 15
   <212> PRT
   <213> INFLUENZA
<400> 19
   Leu Thr Glu Val Glu Thr Pro Ile Arg Asn Glu Trp Gly Cys Arg
   1 5 10 15
<210> 20
   <211> 8
   <212> PRT
   <213> INFLUENZA
<400> 20
   Glu Val Glu Thr Pro Ile Arg Asn
   1 5
<210> 21
   <211> 18
   <212> PRT
   <213> INFLUENZA
<400> 21
   Met Ser Leu Leu Thr Glu Val Glu Thr Pro Thr Arg Asn Glu Trp Glu
   1 5 10 15
   Cys Arg
<210> 22
   <211> 23
   <212> PRT
   <213> INFLUENZA
<400> 22
   Ser Leu Leu Thr Glu Val Glu Thr Pro Thr Arg Asn Glu Trp Glu Cys
   1 5 10 15
   Arg Cys Ser Asp Ser Ser Asp
   20
<210> 23
   <211> 23
   <212> PRT
   <213> INFLUENZA
<400> 23
   Ser Leu Leu Thr Glu Val Glu Thr Pro Ile Arg Asn Glu Trp Gly Cys
   1 5 10 15
   Arg Cys Asn Asp Ser Ser Asp
   20
<210> 24
   <211> 9
   <212> PRT
   <213> INFLUENZA
<400> 24
   Val Glu Thr Pro Ile Arg Asn Glu Trp
   1 5
<210> 25
   <211> 11
   <212> PRT
   <213> INFLUENZA
<400> 25
   Ser Leu Leu Thr Glu Val Glu Thr Tyr Val Leu
   1 5 10
<210> 26
   <211> 11
   <212> PRT
   <213> INFLUENZA
<400> 26
   Ser Leu Leu Thr Glu Val Glu Thr Tyr Val Pro
   1 5 10
<210> 27
   <211> 9
   <212> PRT
   <213> INFLUENZA
<400> 27
   Leu Leu Thr Glu Val Glu Thr Tyr Val
   1 5
<210> 28
   <211> 8
   <212> PRT
   <213> INFLUENZA
<400> 28
   Ser Ile Val Pro Ser Gly Pro Leu
   1 5
<210> 29
   <211> 15
   <212> PRT
   <213> INFLUENZA
<400> 29
   Ser Gly Pro Leu Lys Ala Glu Ile Ala Gln Arg Leu Glu Asp Val
   1 5 10 15
<210> 30
   <211> 12
   <212> PRT
   <213> INFLUENZA
<400> 30
   Gly Pro Leu Lys Ala Glu Ile Ala Gln Arg Leu Glu
   1 5 10
<210> 31
   <211> 9
   <212> PRT
   <213> INFLUENZA
<400> 31
   Arg Leu Glu Asp Val Phe Ala Gly Lys
   1 5
<210> 32
   <211> 11
   <212> PRT
   <213> INFLUENZA
<400> 32
   Ala Leu Met Glu Trp Leu Lys Thr Arg Pro Ile
   1 5 10
<210> 33
   <211> 10
   <212> PRT
   <213> INFLUENZA
<400> 33
   Pro Ile Leu Ser Pro Leu Thr Lys Gly Ile
   1 5 10
<210> 34
   <211> 9
   <212> PRT
   <213> INFLUENZA
<400> 34
   Ile Leu Ser Pro Leu Thr Lys Gly Ile
   1 5
<210> 35
   <211> 19
   <212> PRT
   <213> INFLUENZA
<400> 35
   Leu Thr Lys Gly Ile Leu Gly Phe Val Phe Thr Leu Thr Val Pro Ser
   1 5 10 15
   Glu Arg Gly
<210> 36
   <211> 13
   <212> PRT
   <213> INFLUENZA
<400> 36
   Thr Lys Gly Ile Leu Gly Phe Val Phe Thr Leu Thr Val
   1 5 10
<210> 37
   <211> 12
   <212> PRT
   <213> INFLUENZA
<400> 37
   Lys Gly Ile Leu Gly Phe Val Phe Thr Leu Thr Val
   1 5 10
<210> 38
   <211> 9
   <212> PRT
   <213> INFLUENZA
<400> 38
   Gly Ile Leu Gly Phe Val Phe Thr Leu
   1 5
<210> 39
   <211> 9
   <212> PRT
   <213> INFLUENZA
<400> 39
   Leu Gly Phe Val Phe Thr Leu Thr Val
   1 5
<210> 40
   <211> 9
   <212> PRT
   <213> INFLUENZA
<400> 40
   Ile Leu Gly Phe Val Phe Thr Leu Thr
   1 5
<210> 41
   <211> 8
   <212> PRT
   <213> INFLUENZA
<400> 41
   Ala Ser Cys Met Gly Leu Ile Tyr
   1 5
<210> 42
   <211> 9
   <212> PRT
   <213> INFLUENZA
<400> 42
   Arg Met Gly Ala Val Thr Thr Glu Val
   1 5
<210> 43
   <211> 11
   <212> PRT
   <213> INFLUENZA
<400> 43
   Gly Leu Val Cys Ala Thr Cys Glu Gln Ile Ala
   1 5 10
<210> 44
   <211> 9
   <212> PRT
   <213> INFLUENZA
<400> 44
   Gln Met Val Ala Thr Thr Asn Pro Leu
   1 5
<210> 45
   <211> 10
   <212> PRT
   <213> INFLUENZA
<400> 45
   Gln Met Val Ala Thr Thr Asn Pro Leu Ile
   1 5 10
<210> 46
   <211> 10
   <212> PRT
   <213> INFLUENZA
<400> 46
   Arg Met Val Leu Ala Ser Thr Thr Ala Lys
   1 5 10
<210> 47
   <211> 9
   <212> PRT
   <213> INFLUENZA
<400> 47
   Asp Leu Leu Glu Asn Leu Gln Thr Tyr
   1 5
<210> 48
   <211> 18
   <212> PRT
   <213> INFLUENZA
<400> 48
   Ser Lys Ala Tyr Ser Asn Cys Tyr Pro Tyr Asp Val Pro Asp Tyr Ala
   1 5 10 15
   Ser Leu
<210> 49
   <211> 18
   <212> PRT
   <213> INFLUENZA
<400> 49
   Ser Lys Ala Phe Ser Asn Cys Tyr Pro Tyr Asp Val Pro Asp Tyr Ala
   1 5 10 15
   Ser Leu
<210> 50
   <211> 18
   <212> PRT
   <213> INFLUENZA
<400> 50
   Ser Thr Ala Tyr Ser Asn Cys Tyr Pro Tyr Asp Val Pro Asp Tyr Ala
   1 5 10 15
   Ser Leu
<210> 51
   <211> 10
   <212> PRT
   <213> INFLUENZA
<400> 51
   Trp Leu Thr Glu Lys Glu Gly Ser Tyr Pro
   1 5 10
<210> 52
   <211> 19
   <212> PRT
   <213> INFLUENZA
<400> 52
   Pro Lys Tyr Val Lys Gln Asn Thr Leu Lys Leu Ala Thr Gly Met Arg
   1               5                   10                  15
   Asn Val Pro
<210> 53
   <211> 11
   <212> PRT
   <213> INFLUENZA
<400> 53
   Gly Val Lys Leu Glu Ser Met Gly Ile Tyr Gln
   1               5                   10
<210> 54
   <211> 11
   <212> PRT
   <213> INFLUENZA
<400> 54
   Glu Ile Ser Gly Val Lys Leu Glu Ser Met Gly
   1               5                   10
<210> 55
   <211> 10
   <212> PRT
   <213> INFLUENZA
<400> 55
   Asn Val Lys Asn Leu Tyr Glu Lys Val Lys
   1               5                   10
<210> 56
   <211> 18
   <212> PRT
   <213> INFLUENZA
<400> 56
   Lys Val Lys Ile Leu Pro Lys Asp Arg Trp Thr Gln His Thr Thr Thr
   1               5                   10                  15
   Gly Gly
<210> 57
   <211> 13
   <212> PRT
   <213> INFLUENZA
<400> 57
   Pro Lys Tyr Val Lys Gln Asn Thr Leu Lys Leu Ala Thr
   1               5                   10
<210> 58
   <211> 13
   <212> PRT
   <213> INFLUENZA
<400> 58
   Pro Lys Tyr Val Lys Gln Asn Thr Leu Lys Leu Ala Thr
   1               5                   10
<210> 59
   <211> 9
   <212> PRT
   <213> INFLUENZA
<400> 59
   Cys Thr Glu Leu Lys Leu Ser Asp Tyr
   1               5
<210> 60
   <211> 14
   <212> PRT
   <213> INFLUENZA
<400> 60
   His Pro Ser Ala Gly Lys Asp Pro Lys Lys Thr Gly Gly Pro
   1               5                   10
<210> 61
   <211> 13
   <212> PRT
   <213> INFLUENZA
<400> 61
   His Pro Ser Ala Gly Lys Asp Pro Lys Lys Thr Gly Gly
   1               5                   10
<210> 62
   <211> 24
   <212> PRT
   <213> INFLUENZA
<400> 62
   Phe Trp Arg Gly Glu Asn Gly Arg Lys Thr Arg Ser Ala Tyr Glu Arg
   1 5 10 15
   Met Cys Asn Ile Leu Lys Gly Lys
   20
<210> 63
   <211> 9
   <212> PRT
   <213> INFLUENZA
<400> 63
   Ile Leu Arg Gly Ser Val Ala His Lys
   1 5
<210> 64
   <211> 9
   <212> PRT
   <213> INFLUENZA
<400> 64
   Lys Leu Leu Gln Asn Ser Gln Val Tyr
   1 5
<210> 65
   <211> 15
   <212> PRT
   <213> INFLUENZA
<400> 65
   Ser Ala Ala Phe Glu Asp Leu Arg Val Leu Ser Phe Ile Arg Gly
   1 5 10 15
<210> 66
   <211> 16
   <212> PRT
   <213> INFLUENZA
<400> 66
   Ser Ala Ala Phe Glu Asp Leu Arg Val Ser Ser Phe Ile Arg Gly Thr
   1 5 10 15
<210> 67
   <211> 16
   <212> PRT
   <213> INFLUENZA
<400> 67
   Ser Ala Ala Phe Glu Asp Leu Arg Val Leu Ser Phe Ile Arg Gly Tyr
   1 5 10 15
<210> 68
   <211> 14
   <212> PRT
   <213> INFLUENZA
<400> 68
   Glu Leu Arg Ser Arg Tyr Trp Ala Ile Arg Thr Arg Ser Gly
   1 5 10
<210> 69
   <211> 9
   <212> PRT
   <213> INFLUENZA
<400> 69
   Glu Leu Arg Ser Arg Tyr Trp Ala Ile
   1 5
<210> 70
   <211> 9
   <212> PRT
   <213> INFLUENZA
<400> 70
   Ser Arg Tyr Trp Ala Ile Arg Thr Arg
   1 5
<210> 71
   <211> 10
   <212> PRT
   <213> INFLUENZA
<400> 71
   Tyr Trp Ala Ile Arg Thr Arg Ser Gly Gly
   1 5 10
<210> 72
   <211> 9
   <212> PRT
   <213> INFLUENZA
<400> 72
   Ser Arg Tyr Trp Ala Ile Arg Thr Arg
   1 5
<210> 73
   <211> 9
   <212> PRT
   <213> INFLUENZA
<400> 73
   Leu Pro Phe Asp Lys Pro Thr Ile Met
   1 5
<210> 74
   <211> 9
   <212> PRT
   <213> INFLUENZA
<400> 74
   Val Ser Asp Gly Gly Pro Asn Leu Tyr
   1 5
<210> 75
   <211> 9
   <212> PRT
   <213> INFLUENZA
<400> 75
   Arg Arg Ser Phe Glu Leu Lys Lys Leu
   1 5
<210> 76
   <211> 9
   <212> PRT
   <213> INFLUENZA
<400> 76
   Arg Arg Ala Thr Ala Ile Leu Arg Lys
   1 5
<210> 77
   <211> 9
   <212> PRT
   <213> INFLUENZA
<400> 77
   Arg Pro Ile Ile Arg Pro Ala Thr Leu
   1 5
<210> 78
   <211> 9
   <212> PRT
   <213> INFLUENZA
<400> 78
   Ala Asp Arg Gly Leu Leu Arg Asp Ile
   1 5
<210> 79
   <211> 13
   <212> PRT
   <213> INFLUENZA
<400> 79
   Pro Tyr Tyr Thr Gly Glu His Ala Lys Ala Ile Gly Asn
   1 5 10
<210> 80
   <211> 19
   <212> PRT
   <213> INFLUENZA
<400> 80
   Pro Ala Lys Leu Leu Lys Glu Arg Gly Phe Phe Gly Ala Ile Ala Gly
   1 5 10 15
   Phe Leu Glu
<210> 81
   <211> 12
   <212> DNA
   <213> INFLUENZA
<400> 81
   gaagtggaaa cc 12
<210> 82
   <211> 45
   <212> DNA
   <213> INFLUENZA
<400> 82
   atgagcctgc tgaccgaagt ggaaacccac accaggaatg ggtgg 45
<210> 83
   <211> 27
   <212> DNA
   <213> INFLUENZA
<400> 83
   ccgattcgta acgaatgggg ttgtcgt 27
<210> 84
   <211> 33
   <212> DNA
   <213> INFLUENZA
<400> 84
   gaaaccccga ttcgtaacga atggggttgt cgt 33
<210> 85
   <211> 42
   <212> DNA
   <213> INFLUENZA
<400> 85
   gaaaccccga ttcgtaacga atggggttgt cgtggttgtc gt 42
<210> 86
   <211> 27
   <212> DNA
   <213> INFLUENZA
<400> 86
   ctgctgaccg aagtggaaac cccgatt 27
<210> 87
   <211> 27
   <212> DNA
   <213> INFLUENZA
<400> 87
   agcctgctga ccgaagtgga aaccccg 27
<210> 88
   <211> 30
   <212> DNA
   <213> INFLUENZA
<400> 88
   agcctgctga ccgaagtgga aaccccgatt 30
<210> 89
   <211> 27
   <212> DNA
   <213> INFLUENZA
<400> 89
   ctgaccgaag tggaaacccc gctgacc 27
<210> 90
   <211> 44
   <212> DNA
   <213> INFLUENZA
<400> 90
   tgagcctgct gaccgaagtg gaaaccccga ttcgcaacga atgg 44
<210> 91
   <211> 54
   <212> DNA
   <213> INFLUENZA
<400> 91
   atgagcctgc tgaccgaagt ggaaaccccg attcgcaacg aatggggctg ccgc 54
<210> 92
   <211> 45
   <212> DNA
   <213> INFLUENZA
<400> 92
   atgagcctgc tgaccgaagt ggaaaccctg accaaaaacg gctgg 45
<210> 93
   <211> 45
   <212> DNA
   <213> INFLUENZA
<400> 93
   atgagcctgc tgaccgaagt ggaaaccctg acccgcaacg gctgg 45
<210> 94
   <211> 27
   <212> DNA
   <213> INFLUENZA
<400> 94
   ctgaccgaag tggaaacccc gattcgc 27
<210> 95
   <211> 30
   <212> DNA
   <213> INFLUENZA
<400> 95
   ctgaccgaag tggaaacccc gattcgcaac 30
<210> 96
   <211> 27
   <212> DNA
   <213> INFLUENZA
<400> 96
   gaagtggaaa ccccgattcg caacgaa 27
<210> 97
   <211> 30
   <212> DNA
   <213> INFLUENZA
<400> 97
   gaagtggaaa ccccgattcg caacgaatgg 30
<210> 98
   <211> 33
   <212> DNA
   <213> INFLUENZA
<400> 98
   ctgaccgaag tggaaacccc gattcgcaac gaa 33
<210> 99
   <211> 45
   <212> DNA
   <213> INFLUENZA
<400> 99
   ctgaccgaag tggaaacccc gattcgcaac gaatggggct gccgc 45
<210> 100
   <211> 24
   <212> DNA
   <213> INFLUENZA
<400> 100
   gaagtggaaa ccccgattcg taac 24
<210> 101
   <211> 54
   <212> DNA
   <213> INFLUENZA
<400> 101
   atgagcctgc tgaccgaagt ggaaaccccg acccgcaacg aatgggaatg ccgc 54
<210> 102
   <211> 69
   <212> DNA
   <213> INFLUENZA
<400> 102
   agcctgctga ccgaagtgga aaccccgacc cgcaacgaat gggaatgccg ctgcagcgat 60
   agcagcgat 69
<210> 103
   <211> 69
   <212> DNA
   <213> INFLUENZA
<400> 103
   agcctgctga ccgaagtgga aaccccgatt cgcaacgaat ggggctgccg ctgcaacgat 60
   agcagcgat 69
<210> 104
   <211> 27
   <212> DNA
   <213> INFLUENZA
<400> 104
   gtggaaaccc cgattcgtaa cgaatgg 27
<210> 105
   <211> 33
   <212> DNA
   <213> INFLUENZA
<400> 105
   agcctgctga ccgaagtgga aacctatgtg ctt 33
<210> 106
   <211> 33
   <212> DNA
   <213> INFLUENZA
<400> 106
   agcctgctga ccgaagtgga aacctatgtg ccg 33
<210> 107
   <211> 27
   <212> DNA
   <213> INFLUENZA
<400> 107
   ctgctgaccg aagtggaaac ctatgtg 27
<210> 108
   <211> 24
   <212> DNA
   <213> INFLUENZA
<400> 108
   agcattgtgc cgagcggccc gctg 24
   <210> 109
   <211> 45
   <212> DNA
   <213> INFLUENZA
<400> 109
   agcggcccgc tgaaagcgga aattgcgcag cgcctggaag atgtg 45
<210> 110
   <211> 36
   <212> DNA
   <213> INFLUENZA
<400> 110
   ggcccgctga aagcggaaat tgcgcagcgc ctggaa 36
<210> 111
   <211> 27
   <212> DNA
   <213> INFLUENZA
<400> 111
   cgcctggaag atgtgtttgc gggcaaa 27
<210> 112
   <211> 30
   <212> DNA
   <213> INFLUENZA
<400> 112
   gcgctgatgg aatggctgaa aacccgcccg 30
<210> 113
   <211> 30
   <212> DNA
   <213> INFLUENZA
<400> 113
   ccgattctga gcccgctgac caaaggcatt 30
<210> 114
   <211> 27
   <212> DNA
   <213> INFLUENZA
<400> 114
   attctgagcc cgctgaccaa aggcatt 27
<210> 115
   <211> 57
   <212> DNA
   <213> INFLUENZA
<400> 115
   ctgaccaaag gcattctggg ctttgtgttt accctgaccg tgccgagcga acgcggc 57
<210> 116
   <211> 39
   <212> DNA
   <213> INFLUENZA
<400> 116
   accaaaggca ttctgggctt tgtgtttacc ctgaccgtg 39
<210> 117
   <211> 36
   <212> DNA
   <213> INFLUENZA
<400> 117
   aaaggcattc tgggctttgt gtttaccctg accgtg 36
<210> 118
   <211> 27
   <212> DNA
   <213> INFLUENZA
<400> 118
   ggcattctgg gctttgtgtt taccctg 27
<210> 119
   <211> 27
   <212> DNA
   <213> INFLUENZA
<400> 119
   ctgggctttg tgtttaccct gaccgtg 27
<210> 120
   <211> 27
   <212> DNA
   <213> INFLUENZA
<400> 120
   attctgggct ttgtgtttac cctgacc 27
<210> 121
   <211> 24
   <212> DNA
   <213> INFLUENZA
<400> 121
   gcgagctgca tgggcctgat ttat 24
<210> 122
   <211> 27
   <212> DNA
   <213> INFLUENZA
<400> 122
   cgcatgggcg cggtgaccac cgaagtg 27
<210> 123
   <211> 33
   <212> DNA
   <213> INFLUENZA
<400> 123
   ggcctggtgt gcgcgacctg cgaacagatt gcg 33
<210> 124
   <211> 27
   <212> DNA
   <213> INFLUENZA
<400> 124
   cagatggtgg cgaccaccaa cccgctg 27
<210> 125
   <211> 30
   <212> DNA
   <213> INFLUENZA
<400> 125
   cagatggtgg cgaccaccaa cccgctgatt 30
<210> 126
   <211> 30
   <212> DNA
   <213> INFLUENZA
<400> 126
   cgcatggtgc tggcgagcac caccgcgaaa 30
<210> 127
   <211> 27
   <212> DNA
   <213> INFLUENZA
<400> 127
   gatctgctgg aaaacctgca gacctat 27
<210> 128
   <211> 54
   <212> DNA
   <213> INFLUENZA
<400> 128
   agcaaagctt acagcaactg ttacccttat gatgtgccgg attatgcctc cctt 54
<210> 129
   <211> 54
   <212> DNA
   <213> INFLUENZA
<400> 129
   agcaaagcgt ttagcaactg ctatccgtat gatgtgccgg attatgcgag cctg 54
<210> 130
   <211> 54
   <212> DNA
   <213> INFLUENZA
<400> 130
   agcaccgcgt atagcaactg ctatccgtat gatgtgccgg attatgcgag cctg 54
<210> 131
   <211> 30
   <212> DNA
   <213> INFLUENZA
<400> 131
   tggctgacgg agaaggaggg ctcataccca 30
<210> 132
   <211> 75
   <212> DNA
   <213> INFLUENZA
<400> 132
   cccaagtatg ttaagcaaaa cactctgaag ttggcaacag ggatgcggaa tgtaccagag 60
   aaacaaacta gaggc 75
<210> 133
   <211> 33
   <212> DNA
   <213> INFLUENZA
<400> 133
   ggcgtgaaac tggaaagcat gggcatttat cag 33
<210> 134
   <211> 33
   <212> DNA
   <213> INFLUENZA
<400> 134
   gaaatttccg gcgtgaaact ggaaagcatg ggc 33
<210> 135
   <211> 30
   <212> DNA
   <213> INFLUENZA
<400> 135
   aacgtgaaaa acctgtatga aaaagtgaaa 30
<210> 136
   <211> 54
   <212> DNA
   <213> INFLUENZA
<400> 136
   aaagtgaaaa ttctgccgaa agatcgctgg acccagcata ccaccaccgg cggc 54
<210> 137
   <211> 39
   <212> DNA
   <213> INFLUENZA
<400> 137
   ccgaaatatg tgaaacagaa caccctgaaa ctggcgacc 39
<210> 138
   <211> 39
   <212> DNA
   <213> INFLUENZA
<400> 138
   ccgaaatatg tgaaacagaa caccctgaaa ctggcgacc 39
<210> 139
   <211> 27
   <212> DNA
   <213> INFLUENZA
<400> 139
   tgcaccgaac tgaaactgag cgattat 27
<210> 140
   <211> 42
   <212> DNA
   <213> INFLUENZA
<400> 140
   catccgagcg cgggcaaaga tccgaaaaaa accggcggcc cg 42
<210> 141
   <211> 39
   <212> DNA
   <213> INFLUENZA
<400> 141
   catccgagcg cgggcaaaga tccgaaaaaa accggcggc 39
<210> 142
   <211> 72
   <212> DNA
   <213> INFLUENZA
<400> 142
   ttttggcgcg gcgaaaacgg ccgcaaaacc cgcagcgcgt atgaacgcat gtgcaacatt 60
   ctgaaaggca aa 72
<210> 143
   <211> 27
   <212> DNA
   <213> INFLUENZA
<400> 143
   attctgcgcg gcagcgtggc gcataaa 27
<210> 144
   <211> 27
   <212> DNA
   <213> INFLUENZA
<400> 144
   aaactgctgc agaacagcca ggtgtat 27
<210> 145
   <211> 45
   <212> DNA
   <213> INFLUENZA
<400> 145
   agcgcggcgt ttgaagatct gcgcgtgctg agctttattc gcggc 45
<210> 146
   <211> 48
   <212> DNA
   <213> INFLUENZA
<400> 146
   agcgcggcgt ttgaagatct gcgcgtgagc agctttattc gcggcacc 48
<210> 147
   <211> 48
   <212> DNA
   <213> INFLUENZA
<400> 147
   agcgcggcgt ttgaagatct gcgcgtgctg agctttattc gcggctat 48
<210> 148
   <211> 42
   <212> DNA
   <213> INFLUENZA
<400> 148
   gaactgcgca gccgctattg ggcgattcgc acccgcagcg gc 42
<210> 149
   <211> 27
   <212> DNA
   <213> INFLUENZA
<400> 149
   gaactgcgca gccgctattg ggcgatt 27
<210> 150
   <211> 27
   <212> DNA
   <213> INFLUENZA
<400> 150
   agccgctatt gggcgattcg cacccgc 27
<210> 151
   <211> 30
   <212> DNA
   <213> INFLUENZA
<400> 151
   tattgggcga ttcgcacccg cagcggcggc 30
<210> 152
   <211> 27
   <212> DNA
   <213> INFLUENZA
<400> 152
   agccgctatt gggcgattcg cacccgc 27
<210> 153
   <211> 27
   <212> DNA
   <213> INFLUENZA
<400> 153
   ctgccgtttg ataaaccgac cattatg 27
<210> 154
   <211> 27
   <212> DNA
   <213> INFLUENZA
<400> 154
   gtgagcgatg gcggcccgaa cctgtat 27
<210> 155
   <211> 27
   <212> DNA
   <213> INFLUENZA
<400> 155
   cgccgcagct ttgaactgaa aaaactg 27
<210> 156
   <211> 27
   <212> DNA
   <213> INFLUENZA
<400> 156
   cgccgcgcga ccgcgattct gcgcaaa 27
<210> 157
   <211> 27
   <212> DNA
   <213> INFLUENZA
<400> 157
   cgcccgatta ttcgcccggc gaccctg 27
<210> 158
   <211> 27
   <212> DNA
   <213> INFLUENZA
<400> 158
   gcagatagag ggctattgag agacatc 27
<210> 159
   <211> 39
   <212> DNA
   <213> INFLUENZA
<400> 159
   ccgtattata ccggcgaaca tgcgaaagcg attggcaac 39
<210> 160
   <211> 57
   <212> DNA
   <213> INFLUENZA
<400> 160
   ccggcgaaac tgctgaaaga acgcggcttt tttggcgcga ttgcgggctt tctggaa 57
<210> 161
   <211> 509
   <212> PRT
   <213> salmonella muenchen
<400> 161
   Lys Glu Lys Ile Met Ala Gln Val Ile Asn Thr Asn Ser Leu Ser Leu
   1               5                   10                  15
   Leu Thr Gln Asn Asn Leu Asn Lys Ser Gln Ser Ala Leu Gly Thr Ala
               20                  25                  30
   Ile Glu Arg Leu Ser Ser Gly Leu Arg Ile Asn Ser Ala Lys Asp Asp
           35                  40                  45
   Ala Ala Gly Gln Ala Ile Ala Asn Arg Phe Thr Ala Asn Ile Lys Gly
   50 55 60
   Leu Thr Gln Ala Ser Arg Asn Ala Asn Asp Gly Ile Ser Ile Ala Gln
   65 70 75 80
   Thr Thr Glu Gly Ala Leu Asn Glu Ile Asn Asn Asn Leu Gln Arg Val
   85 90 95
   Arg Glu Leu Ala Val Gln Ser Ala Asn Gly Thr Asn Ser Gln Ser Asp
   100 105 110
   Leu Asp Ser Ile Gln Ala Glu Ile Thr Gln Arg Leu Asn Glu Ile Asp
   115 120 125
   Arg Val Ser Gly Gln Thr Gln Phe Asn Gly Val Lys Val Leu Ala Gln
   130 135 140
   Asp Asn Thr Leu Thr Ile Gln Val Gly Ala Asn Asp Gly Glu Thr Ile
   145 150 155 160
   Asp Ile Asp Leu Lys Glu Ile Ser Ser Lys Thr Leu Gly Leu Asp Lys
   165 170 175
   Leu Asn Val Gln Asp Ala Tyr Thr Pro Lys Glu Thr Ala Val Thr Val
   180 185 190
   Asp Lys Thr Thr Tyr Lys Asn Gly Thr Asp Thr Ile Thr Ala Gln Ser
   195 200 205
   Asn Thr Asp Ile Gln Thr Ala Ile Gly Gly Gly Ala Thr Gly Val Thr
   210 215 220
   Gly Ala Asp Ile Lys Phe Lys Asp Gly Gln Tyr Tyr Leu Asp Val Lys
   225 230 235 240
   Gly Gly Ala Ser Ala Gly Val Tyr Lys Ala Thr Tyr Asp Glu Thr Thr
   245 250 255
   Lys Lys Val Asn Ile Asp Thr Thr Asp Lys Thr Pro Leu Ala Thr Ala
   260 265 270
   Glu Ala Thr Ala Ile Arg Gly Thr Ala Thr Ile Thr His Asn Gln Ile
   275 280 285
   Ala Glu Val Thr Lys Glu Gly Val Asp Thr Thr Thr Val Ala Ala Gln
   290 295 300
   Leu Ala Ala Ala Gly Val Thr Gly Ala Asp Lys Asp Asn Thr Ser Leu
   305 310 315 320
   Val Lys Leu Ser Phe Glu Asp Lys Asn Gly Lys Val Ile Asp Gly Gly
   325 330 335
   Tyr Ala Val Lys Met Gly Asp Asp Phe Tyr Ala Ala Thr Tyr Asp Glu
   340 345 350
   Lys Gln Val Gln Leu Leu Leu Asn Asn His Tyr Thr Asp Gly Ala Gly
   355 360 365
   Val Leu Gln Thr Gly Ala Val Lys Phe Gly Gly Ala Asn Gly Lys Ser
   370 375 380
   Glu Val Val Thr Ala Thr Val Gly Lys Thr Tyr Leu Ala Ser Asp Leu
   385 390 395 400
   Asp Lys His Asn Phe Arg Thr Gly Gly Glu Leu Lys Glu Val Asn Thr
   405 410 415
   Asp Lys Thr Glu Asn Pro Leu Gln Lys Ile Asp Ala Ala Leu Ala Gln
   420 425 430
   Val Asp Thr Leu Arg Ser Asp Leu Gly Ala Val Gln Asn Arg Phe Asn
   435 440 445
   Ser Ala Ile Thr Asn Leu Gly Asn Thr Val Asn Asn Leu Ser Ser Ala
   450 455 460
   Arg Ser Arg Ile Glu Asp Ser Asp Tyr Ala Thr Glu Val Ser Asn Met
   465 470 475 480
   Ser Arg Ala Gln Ile Leu Gln Gln Ala Gly Thr Ser Val Leu Ala Gln
   485 490 495
   Ala Asn Gln Val Pro Gln Asn Val Leu Ser Leu Leu Arg
   500 505
<210> 162
   <211> 1530
   <212> DNA
   <213> salmonella muenchen
<400> 162
   aaggaaaaga tcatggcaca agtcattaat acaaacagcc tgtcgctgtt gacccagaat     60
   aacctgaaca aatcccagtc cgctctgggc accgctatcg agcgtctgtc ttccggtctg    120
   cgtatcaaca gcgcgaaaga cgatgcggca ggtcaggcga ttgctaaccg tttcaccgcg    180
   aacatcaaag gtctgactca ggcttcccgt aacgctaacg acggtatctc cattgcgcag    240
   accactgaag gcgcgctgaa cgaaatcaac aacaacctgc agcgtgtgcg tgaactggcg    300
   gttcagtctg ctaacggtac taactcccag tctgaccttg actctatcca ggctgaaatc    360
   acccagcgtc tgaacgaaat cgaccgtgta tccggtcaga ctcagttcaa cggcgtgaaa    420
   gtcctggcgc aggacaacac cctgaccatc caggttggtg ccaacgacgg tgaaactatt    480
   gatattgatt taaaagaaat tagctctaaa acactgggac ttgataagct taatgtccag    540
   gatgcctaca ccccgaaaga aactgctgta accgttgata aaactaccta taaaaatggt    600
   acagatacta ttacagccca gagcaatact gatatccaaa ctgcaattgg cggtggtgca    660
   acgggggtta ctggggctga tatcaaattt aaagatggtc aatactattt agatgttaaa    720
   ggcggtgctt ctgctggtgt ttataaagcc acttatgatg aaactacaaa gaaagttaat    780
   attgatacga ctgataaaac tccgttagca actgcggaag ctacagctat tcggggaacg    840
   gccactataa cccacaacca aattgctgaa gtaacaaaag agggtgttga tacgaccaca    900
   gttgcggctc aacttgctgc tgcaggggtt actggtgccg ataaggacaa tactagcctt    960
   gtaaaactat cgtttgagga taaaaacggt aaggttattg atggtggcta tgcagtgaaa   1020
   atgggcgacg atttctatgc cgctacatat gatgagaaac aggtacaatt actgctaaac   1080
   aaccactata cagatggtgc tggcgtgctc caaactggag ctgtgaaatt tggtggcgca   1140
   aatggtaaat ctgaagttgt tactgctacc gtaggtaaaa cttacttagc aagcgacctt   1200
   gacaaacata acttcagaac aggcggtgag cttaaagagg ttaatacaga taagactgaa   1260
   aacccactgc agaaaattga tgctgccttg gcacaggttg atacacttcg ttctgacctg   1320
   ggtgcggtac agaaccgttt caactccgct atcaccaacc tgggcaatac cgtaaataac   1380
   ctgtcttctg cccgtagccg tatcgaagat tccgactacg cgaccgaagt ctccaacatg   1440
   tctcgcgcgc agattctgca gcaggccggt acctccgttc tggcgcaggc taaccaggtt   1500
   ccgcaaaacg tcctctcttt actgcgttaa                                    1530